# EUROPEAN PATENT APPLICATION

(11) **EP 3 604 508 A1**
(43) Date of publication of application: **05.02.2020**
(21) Application number: 18770777.3
(22) Date of filing: 23.03.2018
(51) Int. Cl.: C12N 9/58, C12N 1/19, C12N 15/09, C12P 21/02

(54) **METHOD FOR PRODUCING PROTEASE IN YEAST**

(30) Priority: 24.03.2017 JP 2017058983
(71) Applicant: Kaneka Corporation, Osaka-shi, Osaka 530-8288 (JP)
(72) Inventor: NISHIYAMA Tozo, Takasago-shi Hyogo 676-8688 (JP)
(74) Representative: Hoffmann Eitle
(86) International application number: PCT/JP2018/011631
(87) International publication number: WO 2018/174231

(57) **Abstract**

It is an object of the present invention to provide a method for producing a mold-derived trypsin-like protease by using yeast as a host, by which the mold-derived trypsin-like protease can be produced with high productivity. The present invention provides a method for producing a protease in yeast, which comprises expressing a gene encoding a fusion protein having a secretion signal sequence functioning in yeast, a pro-sequence of a *Fusarium oxysporum*-derived trypsin-like protease, into which a mutation is introduced and which has an arginine or lysine residue at the C-terminus thereof, and the amino acid sequence of the *Fusarium oxysporum*-derived trypsin-like protease, in this order from the N-terminal side to C-terminal side thereof, in yeast.

## Description

### Technical Field

The present invention relates to a method for producing a mold-derived trypsin-like protease in yeast, a mold-derived trypsin-like protease precursor, and the like.

### Background Art

In order to produce a protein according to a genetic recombination technique, a host suitable for the expression of the protein is used. Examples of the host used herein may include: animal cells such as CHO (Chinese Hamster Ovary) cells; insects and insect cells, such as silk worms; animals such as chickens or bovines; and microorganisms such as *Escherichia coli* or yeast. Among the above-described hosts, yeast enables a large-scale high-density culture in an inexpensive medium, and thus, using such yeast, proteins can be produced at low costs. In addition, if a secretion signal peptide and the like are used, it becomes possible to perform secretory production of proteins into a culture medium. Hence, it becomes easy to purify proteins.

Trypsin, which is a serine protease produced in the pancreas of mammals, has a protease activity of cleaving the C-terminal side of an arginine residue or a lysine residue, and is used in production of proteinous pharmaceutical products such as insulin, cell dispersion, and the like. Other than trypsin derived from mammals (bovines, swines and humans), a trypsin-like protease derived from mold or fish has been known.

For example, Patent Document 1 discloses purification of a trypsin-like protease from the Fusarium mold (*Fusarium oxysporum*) and the cloning of the trypsin-like protease gene. Non-Patent Document 1 discloses production of a trypsin-like protease in the Fusarium mold (*Fusarium oxysporum*)*.*

Moreover, Non-Patent Document 2 discloses the expression of *Streptomyces griseus*-derived trypsin in the yeast (*Pichia pastosis*)*.* Furthermore, Non-Patent Document 3 discloses the expression of swine trypsin in the yeast (*Pichia pastosis*)*.*

### Prior Art Documents

### Patent Documents

Patent Document 1: International Publication WO1994/025583
Non-Patent Document 1: Natalie E. Farnworth et al., Enzyme and Microbial Technology 33 (2003) 85-91
Non-Patent Document 2: Zhenmin Ling et al., J Ind Microbiol Biotechnol (2012) 39:1651-1662
Non-Patent Document 3: Min Shu et al., Protein Expression and Purification 114 (2015) 149-155

### Summary of Invention

### Object to be Solved by the Invention

It is an object of the present invention to provide a method for producing a mold-derived trypsin-like protease by using yeast as a host, by which the mold-derived trypsin-like protease can be produced with high productivity.

### Means for Solving the Object

As a result of intensive studies directed towards achieving the aforementioned object, the present inventors found that a gene encoding a fusion protein having a secretion signal sequence functioning in yeast, a pro-sequence possessed by a *Fusarium oxysporum-derived* trypsin-like protease, into which a mutation has been introduced, and the amino acid sequence of the *Fusarium oxysporum-derived* trypsin-like protease, in this order from the N-terminal side to the C-terminal side thereof, is expressed in yeast, so that the *Fusarium oxysporum*-derived trypsin-like protease can be produced with high productivity, thereby completing the present invention.

Specifically, according to the present invention, the following inventions are provided.
(1) A method for producing a protease in yeast, which comprises expressing a gene encoding a fusion protein having a secretion signal sequence functioning in yeast, a pro-sequence of a *Fusarium oxysporum-derived* trypsin-like protease, into which a mutation is introduced and which has an arginine or lysine residue at the C-terminus thereof, and the amino acid sequence of the *Fusarium oxysporum-derived* trypsin-like protease, in this order from the N-terminal side to C-terminal side thereof, in yeast.
(2) The method according to the above (1), wherein the protease, and a protease precursor consisting of the pro-sequence and the amino acid sequence of the protease, are secreted at an activity ratio of 1 : 1 or more in the culture supernatant of the yeast.
(3) The method according to the above (1) or (2), wherein the protease precursor expressed in the culture supernatant is converted to a protease by the protease expressed in the culture supernatant.
(4) The method according to any one of the above (1) to (3), wherein the *Fusarium oxysporum-derived* trypsin-like protease is any one of the following (a) to (c):
   (a) a protease consisting of the amino acid sequence as set forth in SEQ ID NO: 67;
   (b) a protease consisting of an amino acid sequence having a sequence identity of 90% or more to the amino acid sequence as set forth in SEQ ID NO: 67, and having a trypsin-like protease activity equivalent to that of the protease consisting of the amino acid sequence as set forth in SEQ ID NO: 67; or
   (c) a protease consisting of an amino acid sequence comprising a substitution, deletion and/or addition of one or several amino acids with respect to the amino acid sequence as set forth in SEQ ID NO: 67, and having a trypsin-like protease activity equivalent to that of the protease consisting of the amino acid sequence as set forth in SEQ ID NO: 67.
(5) The method according to any one of the above (1) to (4), wherein the secretion signal sequence functioning in yeast is a yeast MF sequence.
(6) The method according to any one of the above (1) to (5), wherein the pro-sequence is an amino acid sequence comprising an addition, deletion or substitution of one or several amino acids with respect to the amino acid sequence shown in Ala-Pro-Gln-Glu-Ile-Pro-Asn, and the amino acid sequence has an arginine or lysine residue at the C-terminus thereof, and is cleaved at the C-terminus thereof in yeast, so that a protease is generated from the protease precursor.
(7) The method according to any one of the above (1) to (6), wherein the pro-sequence is an amino acid sequence shown in any one of the following:
   Ala- Pro-Gln-Glu-Ile-Pro-Xaa-Xbb,
   Xcc-Ala- Pro-Gln-Glu-Ile-Pro-Xaa-Xbb,
   Xdd-Xcc-Ala- Pro-Gln-Glu-Ile-Pro-Xaa-Xbb,
   Pro-Gln-Glu-Ile-Pro-Xaa-Xbb,
   Gln-Glu-Ile-Pro-Xaa-Xbb,
   Glu-Ile-Pro-Xaa-Xbb,
   Ile-Pro-Xaa-Xbb,
   Pro-Xaa-Xbb, or
   Xaa-Xbb,
   wherein Xaa represents any given amino acid residue, Xbb represents an Arg or Lys residue,
   Xcc represents any given amino acid residue, and Xdd represents any given amino acid residue.
(8) A protease precursor, in which a pro-sequence having an arginine or lysine residue at the C-terminus thereof is fused to the N-terminal side of the amino acid sequence of the *Fusarium oxysporum-derived* trypsin-like protease, wherein
   (i) when the protease precursor is expressed in the culture supernatant of yeast, the protease, and a protease precursor consisting of the pro-sequence and the amino acid sequence of the protease, are secreted at an activity ratio of 1 : 1 or more in the culture supernatant of the yeast, and
   (ii) the expressed protease precursor can be converted to a protease by the expressed protease.
(9) The protease precursor according to the above (8), wherein the *Fusarium oxysporum-derived* trypsin-like protease is any one of the following (a) to (c):
   (a) a protease consisting of the amino acid sequence as set forth in SEQ ID NO: 67;
   (b) a protease consisting of an amino acid sequence having a sequence identity of 90% or more to the amino acid sequence as set forth in SEQ ID NO: 67, and having a trypsin-like protease activity equivalent to that of the protease consisting of the amino acid sequence as set forth in SEQ ID NO: 67; or
   (c) a protease consisting of an amino acid sequence comprising a substitution, deletion and/or addition of one or several amino acids with respect to the amino acid sequence as set forth in SEQ ID NO: 67, and having a trypsin-like protease activity equivalent to that of the protease consisting of the amino acid sequence as set forth in SEQ ID NO: 67.
(10) The protease precursor according to the above (8) or (9), wherein the pro-sequence is an amino acid sequence comprising an addition, deletion or substitution of one or several amino acids with respect to the amino acid sequence shown in Ala-Pro-Gln-Glu-Ile-Pro-Asn, and the amino acid sequence has an arginine or lysine residue at the C-terminus thereof, and is cleaved at the C-terminus thereof in yeast, so that a protease is generated from the protease precursor.
(11) The protease precursor according to any one of the above (8) to (10), wherein the pro-sequence is an amino acid sequence shown in any one of the following:
   Ala- Pro-Gln-Glu-Ile-Pro-Xaa-Xbb,
   Xcc-Ala- Pro-Gln-Glu-Ile-Pro-Xaa-Xbb,
   Xdd-Xcc-Ala- Pro-Gln-Glu-Ile-Pro-Xaa-Xbb,
   Pro-Gln-Glu-Ile-Pro-Xaa-Xbb,
   Gln-Glu-Ile-Pro-Xaa-Xbb,
   Glu-Ile-Pro-Xaa-Xbb,
   Ile-Pro-Xaa-Xbb,
   Pro-Xaa-Xbb, or
   Xaa-Xbb,
   wherein Xaa represents any given amino acid residue, Xbb represents an Arg or Lys residue,
   Xcc represents any given amino acid residue, and Xdd represents any given amino acid residue.
(12) A recombinant expression vector having DNA that encodes a fusion protein having a secretion signal sequence functioning in yeast, a pro-sequence of a *Fusarium oxysporum-derived* trypsin-like protease, into which a mutation is introduced and which has an arginine or lysine residue at the C-terminus thereof, and the amino acid sequence of the *Fusarium oxysporum-derived* trypsin-like protease, in this order from the N-terminal side to C-terminal side thereof.
(13) The recombinant expression vector according to the above (12), wherein the *Fusarium oxysporum-derived* trypsin-like protease is any one of the following (a) to (c):
   (a) a protease consisting of the amino acid sequence as set forth in SEQ ID NO: 67;
   (b) a protease consisting of an amino acid sequence having a sequence identity of 90% or more to the amino acid sequence as set forth in SEQ ID NO: 67, and having a trypsin-like protease activity equivalent to that of the protease consisting of the amino acid sequence as set forth in SEQ ID NO: 67; or
   (c) a protease consisting of an amino acid sequence comprising a substitution, deletion and/or addition of one or several amino acids with respect to the amino acid sequence as set forth in SEQ ID NO: 67, and having a trypsin-like protease activity equivalent to that of the protease consisting of the amino acid sequence as set forth in SEQ ID NO: 67.
(14) The recombinant expression vector according to the above (12) or (13), wherein the secretion signal sequence functioning in yeast is a yeast MF sequence.
(15) The recombinant expression vector according to any one of the above (12) to (14), wherein the pro-sequence is an amino acid sequence comprising an addition, deletion or substitution of one or several amino acids with respect to the amino acid sequence shown in Ala- Pro-Gln-Glu-Ile-Pro-Asn, and the amino acid sequence is cleaved at the C-terminus thereof in yeast, so that a protease is generated from the protease precursor.
(16) The recombinant expression vector according to any one of the above (12) to (15), wherein the pro-sequence is an amino acid sequence shown in any one of the following:
   Ala- Pro-Gln-Glu-Ile-Pro-Xaa-Xbb,
   Xcc-Ala- Pro-Gln-Glu-Ile-Pro-Xaa-Xbb,
   Xdd-Xcc-Ala- Pro-Gln-Glu-Ile-Pro-Xaa-Xbb,
   Pro-Gln-Glu-Ile-Pro-Xaa-Xbb,
   Gln-Glu-Ile-Pro-Xaa-Xbb,
   Glu-Ile-Pro-Xaa-Xbb,
   Ile-Pro-Xaa-Xbb,
   Pro-Xaa-Xbb, or
   Xaa-Xbb,
   wherein Xaa represents any given amino acid residue, Xbb represents an Arg or Lys residue,
   Xcc represents any given amino acid residue, and Xdd represents any given amino acid residue.
(17) A transformed yeast having the recombinant expression vector according to any one of the above (12) to (16).

Further, according to the present invention, the following inventions are provided.
(A) A yeast culture-derived protease composition comprising a *Fusarium oxysporum-derived* trypsin-like protease that is expressed in yeast.
(B) The yeast culture-derived protease composition according to the above (A),
   wherein the *Fusarium oxysporum-derived* trypsin-like protease is any one of the following (a) to (c):
   (a) a protease consisting of the amino acid sequence as set forth in SEQ ID NO: 67;
   (b) a protease consisting of an amino acid sequence having a sequence identity of 90% or more to the amino acid sequence as set forth in SEQ ID NO: 67, and having a trypsin-like protease activity equivalent to that of the protease consisting of the amino acid sequence as set forth in SEQ ID NO: 67; or
   (c) a protease consisting of an amino acid sequence comprising a substitution, deletion and/or addition of one or several amino acids with respect to the amino acid sequence as set forth in SEQ ID NO: 67, and having a trypsin-like protease activity equivalent to that of the protease consisting of the amino acid sequence as set forth in SEQ ID NO: 67.

### Advantageous Effects of Invention

According to the present invention, a mold-derived trypsin-like protease can be produced with high productivity in yeast. In the method for producing a mold-derived trypsin-like protease according to the present invention, yeast enabling high-density culture can be used as a host, and thus, the production cost of the mold-derived trypsin-like protease can be reduced.

### Brief Description of Drawings

[Fig.1] Fig. 1 shows the results of the SDS-PAGE analysis of the culture supernatant obtained in Comparative Example 3. Lane 1 indicates MF-pro-sequence-protease, and Lane 2 indicates MF-protease.
[Fig.2] Fig. 2 shows the results of the SDS-PAGE analysis of the culture supernatant obtained in Example 6, before and after performing an autodigestion treatment on the sample. M indicates a marker; Lane 1 indicates a culture supernatant (MF-APQEIPNK-trypsin) before a conversion reaction; and Lane 2 indicates the results after an autodigestion reaction.

### Embodiment of Carrying out the Invention

Hereinafter, the embodiments of the present invention will be described in more details.

### [Production of protease in yeast]

In the present invention, a gene encoding a fusion protein having a secretion signal sequence functioning in yeast, a pro-sequence (more specifically, a pro-sequence of a *Fusarium oxysporum*-derived trypsin-like protease, into which a mutation is introduced and which has an arginine or lysine residue at the C-terminus thereof), and the amino acid sequence of the *Fusarium oxysporum*-derived trypsin-like protease, in this order from the N-terminal side to C-terminal side thereof, is expressed in yeast, so that a protease is produced in the yeast.

In the present invention, the above-described fusion protein is expressed in yeast by using a recombinant expression vector having DNA that encodes the secretion signal sequence functioning in yeast, the above-described pro-sequence, and the amino acid sequence of the *Fusarium oxysporum*-derived trypsin-like protease, so that a protease precursor consisting of the pro-sequence and the amino acid sequence of the protease, and the protease, are first expressed in the culture supernatant of the yeast. Moreover, the present inventors found that the protease precursor expressed in the culture supernatant is converted to a protease by the protease expressed in the culture supernatant. According to the above-described mechanism, in the present invention, it became possible to efficiently produce (manufacture) a *Fusarium oxysporum*-derived trypsin-like protease having a desired protease activity in yeast.

The expression ratio between the protease and the protease precursor in the culture supernatant of the yeast (i.e., the expression ratio before an autodigestion reaction) is not particularly limited, but the protease and the protease precursor are secreted into the culture supernatant at an activity ratio of preferably 1 : 1 or more, more preferably 1 : 10 or more, and further preferably 1 : 100 or more.

The term "activity ratio" is used herein to mean an expression level ratio, which is obtained when the amount of a protease activity in a culture supernatant (i.e., the amount of a protease activity in a culture supernatant, before the conversion of a protease precursor to a protease due to autodigestion after the culture) is defined as the expression level of a protease, and also when the activity amount obtained by subtracting the amount of a protease activity in a culture supernatant from the amount of a protease activity after the autodigestion is defined as the expression level of a protease precursor.

### [Fusarium oxysporum-derived trypsin-like protease]

The "*Fusarium oxysporum*-derived trypsin-like protease" of the present invention means a trypsin-like protease expressed by *Fusarium oxysporum,* or a modified form thereof. The trypsin-like protease expressed by *Fusarium oxysporum* is a protease consisting of the amino acid sequence as set forth in SEQ ID NO: 67.

In the present invention, as a modified form of the trypsin-like protease expressed by *Fusarium oxysporum,* the following protease may be expressed:
(b) a protease consisting of an amino acid sequence having a sequence identity of 90% or more (preferably 95% or more, and more preferably 98% or more) to the amino acid sequence as set forth in SEQ ID NO: 67, and having a trypsin-like protease activity equivalent to that of the protease consisting of the amino acid sequence as set forth in SEQ ID NO: 67; or
(c) a protease consisting of an amino acid sequence comprising a substitution, deletion and/or addition of one or several (preferably 1 to 10, more preferably 1 to 5, and further preferably 1 to 3) amino acids with respect to the amino acid sequence as set forth in SEQ ID NO: 67, and having a trypsin-like protease activity equivalent to that of the protease consisting of the amino acid sequence as set forth in SEQ ID NO: 67. Examples of such proteases may include mutants described in Examples 9 and 10, in which R104 residue (i.e., the amino acid residue at position 104 that is an Arg residue) in SEQ ID NO: 67 is substituted with another amino acid.

The sequence identity of amino acid sequences can be determined by using the algorithms according to Karlin and Altschul, BLAST [Pro. Natl. Acad. Sci. USA, 90, 5873 (1993)] or FASTA [Methods Enzymol., 183, 63 (1990)].

Moreover, in the case of substitution of amino acids, the substituting amino acid is preferably an amino acid having properties similar to those of the amino acid before the substitution (i.e., homologous amino acid). Herein, the amino acids in each group as shown below are defined as homologous amino acids:
(First group: neutral, non-polar amino acids) Gly, Ala, Val, Leu, Ile, Met, Cys, Pro, and Phe;
(Second group: neutral, polar amino acids) Ser, Thr, Gln, Asn, Trp, and Tyr;
(Third group: acidic amino acids) Glu and Asp; and
(Fourth group: basic amino acids) His, Lys, and Arg.

DNA encoding the above-described modified form of the trypsin-like protease can be obtained by common methods known to a person skilled in the art, such as site-directed mutagenesis, and the DNA can also be obtained by using, for example, a commercially available mutation introduction kit (e.g., Mutant-K, Mutant-G, LA PCR *in vitro* Mutagenesis series kit (manufactured by TAKARA)):

### [Secretion signal sequence functioning in yeast]

The secretion signal sequence functioning in yeast used in the present invention is a sequence that commands the transport of proteins biosynthesized in the cytoplasm into the endoplasmic reticulum, and the localization thereof. The secretion signal sequence is a peptide that exists at the N-terminus of a protein, so that it has the function of secreting the expressed protein to the outside of yeast as a host cell. In general, the secretion signal sequence is removed by being decomposed by signal peptidase when a secretory protein is secreted from a cell to the outside of the cell through the cell membrane.

The secretion signal sequence functioning in yeast, which is used in the present invention, is not particularly limited, as long as it may be a sequence functioning in yeast. The secretion signal sequence functioning in yeast may be either a yeast-derived secretion signal sequence, or a secretion signal sequence derived from organisms other than yeast. It is preferably a yeast-derived secretion signal sequence.

Examples of the secretion signal sequence functioning in yeast may include the Mating Factor α (MFα) prepro signal sequence (MF sequence) of yeast (*Saccharomyces cerevisiae,* etc.), and the signal sequences of acid phosphatase (PHO1) of *Ogataea* *polymorpha* or *Komagataella pastoris,* invertase (SUC2) of *Saccharomyces cerevisiae,* and PLB1 of *Saccharomyces cerevisiae,* but are not particularly limited thereto.

### [Pro-sequence]

The pro-sequence used in the present invention is obtained by introducing a mutation into the pro-sequence of a *Fusarium oxysporum*-derived trypsin-like protease, and the present pro-sequence preferably has an arginine or lysine residue at the C-terminus thereof.

The amino acid sequence of the pro-sequence of the *Fusarium oxysporum*-derived trypsin-like protease may be, for example, Ala- Pro-Gln-Glu-Ile-Pro-Asn. Accordingly, the pro-sequence used in the present invention is an amino acid sequence comprising an addition deletion or substitution of one to several (preferably 1 to 7, more preferably 1 to 6, and for example, 1, 2, 3, 4, 5 or 6) amino acids, with respect to the amino acid sequence shown in Ala- Pro-Gln-Glu-Ile-Pro-Asn, and having an arginine or lysine residue at the C-terminus thereof.

The pro-sequence used in the present invention is an amino acid sequence that is cleaved at the C-terminus thereof in yeast, so that a protease is generated from a protease precursor. In the case of substitution of amino acids, the substituting amino acid is preferably an amino acid having properties similar to those of the amino acid before the substitution (i.e., homologous amino acid). The homologous amino acids are as described above in the present description.

A specific example of the pro-sequence may be an amino acid sequence shown in any one of the following:
Ala- Pro-Gln-Glu-Ile-Pro-Xaa-Xbb,
Xcc-Ala- Pro-Gln-Glu-Ile-Pro-Xaa-Xbb,
Xdd-Xcc-Ala- Pro-Gln-Glu-Ile-Pro-Xaa-Xbb,
Pro-Gln-Glu-Ile-Pro-Xaa-Xbb,
Gln-Glu-Ile-Pro-Xaa-Xbb,
Glu-Ile-Pro-Xaa-Xbb,
Ile-Pro-Xaa-Xbb,
Pro-Xaa-Xbb, or
Xaa-Xbb,
wherein Xaa represents any given amino acid residue, Xbb represents an Arg or Lys residue,
Xcc represents any given amino acid residue, and Xdd represents any given amino acid residue.

Other specific examples of the pro-sequence may include: an amino acid sequence comprising a substitution of one to several (preferably 1 to 7, more preferably 1 to 6, and for example 1, 2, 3, 4, 5 or 6) amino acids, with respect to the amino acid sequence shown in Ala- Pro-Gln-Glu-Ile-Pro-Asn-Arg; and an amino acid sequence comprising a substitution of one to several (preferably 1 to 7, more preferably 1 to 6, and for example 1, 2, 3, 4, 5 or 6) amino acids, with respect to the amino acid sequence shown in Ala-Pro-Gln-Glu-Ile-Pro-Asn-Lys.

Xaa, Xbb and Xcc each independently represent any given amino acid residue, and specifically represent any one of Asp, Gly, Ala, Val, Leu, Ile, Cys, Met, Ser, Thr, Tyr, Phe, Trp, Pro, Glu, Asn, Gln, Lys, Arg, and His. It is to be noted that Asp, Gly, Ala, Val, Leu, Ile, Cys, Met, Ser, Thr, Tyr, Phe, Trp, Pro, Glu, Asn, Gln, Lys, Arg, and His indicate, respectively, an aspartic acid residue, a glycine residue, an alanine residue, a valine residue, a leucine residue, an isoleucine residue, a cysteine residue, a methionine residue, a serine residue, a threonine residue, a tyrosine residue, a phenylalanine residue, a tryptophan residue, a proline residue, a glutamic acid residue, an asparagine residue, a glutamine residue, a lysine residue, an arginine residue, and a histidine residue.

Xaa is preferably Asn, Gly, Asp, Ser, Arg, Thr, Glu, Val, His, Ala, Tyr, Ile, Gln, or Met.

Xcc and Xdd are preferably neutral amino acid residues (Gly, Ala, Val, Leu, Ile, Cys, Met, Ser, Thr, Tyr, Phe, Trp, Pro, Asn, or Gln), more preferably aliphatic amino acid residues (Gly, Ala, Val, Leu, or Ile), and further preferably Ala.

### [Recombinant expression vector]

In the present invention, a recombinant expression vector having DNA that encodes a fusion protein having a secretion signal sequence functioning in yeast, a pro-sequence of a *Fusarium oxysporum*-derived trypsin-like protease, into which a mutation is introduced and which has an arginine or lysine residue at the C-terminus thereof, and the amino acid sequence of the *Fusarium oxysporum*-derived trypsin-like protease, in this order from the N-terminal side to C-terminal side thereof, is used to express and secrete a *Fusarium oxysporum*-derived trypsin-like protease in yeast.

The term "recombinant expression vector" is used in the present invention to mean a nucleic acid molecule having the function of expressing a gene in an expression cassette incorporated into the recombinant expression vector in a host cell after transformation. The recombinant expression vector may have a homologous region for incorporation, a selective marker gene such as an auxotrophic complementary gene or a drug resistance gene, an autonomous replicating sequence, and the like, in addition to the expression cassette.

In the present invention, the vector after transformation into a host may be integrated into the chromosome of the transformant, or may also be present as an autonomous replicating vector.

The expression vector may also be a plasmid vector or an artificial chromosome. From the viewpoint of easy preparation of a vector and easy transformation of yeast cells, a plasmid vector is preferable. Examples of the plasmid may include *Escherichia coli*-derived plasmids (e.g., pBR322, pBR325, pUC118, pUC119, pUC18, pUC19, pBluescript, etc.), *Bacillus subtilis*-derived plasmids (e.g., pUB110, pTP5, etc.), and yeast-derived plasmids (e.g., YEp systems such as YEp13, YCp systems such as YCp50, etc.), but are not particularly limited thereto.

The "expression cassette" used in the present invention is composed of a promoter and a desired protein to be expressed, and the expression cassette may also comprise a terminator. The expression cassette may be constituted on a plasmid such as, for example, pUC, or may also be produced according to a PCR method.

Examples of the promoter that can be used herein may include an AOX1 promoter, an AOX2 promoter, a CAT promoter, a DHAS promoter, an FDH promoter, an FMD promoter, a GAP promoter, a MOX promoter, a TEF promoter, a LEU2 promoter, a URA3 promoter, an ADE promoter, an ADH1 promoter, and a PGK1 promoter, but are not particularly limited thereto.

Examples of the terminator that can be used herein may include an AOX1 terminator, a GAP terminator, and an ADH1 terminator, but are not particularly limited thereto.

The homologous region for integration, which is used in the present invention, indicates a region in which the recombinant vector of the present invention is to be integrated into the chromosome of a transformed host cell according to homologous recombination. As such a region, a portion of the chromosome of a host cell can be arbitrarily used, and further, an auxotrophic complementary gene, or a promoter, a terminator or the like comprised in the expression cassette, can also be used.

The auxotrophic complementary gene used in the present invention is not particularly limited, as long as it is a gene that complements the nutritional requirements of host cells for amino acids, nucleic acids, and the like. Specific examples of the auxotrophic complementary gene may include a URA3 gene, a LEU2 gene, an ADE1 gene, and a HIS4 gene. The URA3 gene, LEU2 gene, ADE1 gene, and HIS4 gene can be selected by the recovery of the phenotypes of auxotrophic strains for uracil, leucine, adenine, and histidine, respectively.

The selective marker gene used in the present invention, such as a drug resistance gene, is not particularly limited, as long as it is a gene imparting to a host cell, drug resistance that has not been possessed by the host cell. Specific examples of such a selective marker gene may include a G418 resistance gene, a Zeocin resistance gene, and a hygromycin resistance gene. These genes can be selected depending on resistance on a medium comprising G418, Zeocin, or hygromycin, respectively. Besides, the auxotrophic selective marker that has been used in the production of a yeast host cannot be used, unless the selective marker is destructed. In this case, it is appropriate if the selective marker is recovered. As a method of recovering the selective marker, a method known to a person skilled in the art can be used.

The term "autonomous replicating sequence" is used in the present invention to mean a sequence acting as a replication origin of the recombinant vector of the present invention in host cells, and being capable of autonomous replication.

### [Transformed yeast]

According to the present invention, provided is a transformed yeast having the above-described recombinant expression vector of the present invention.

The type of the yeast is not particularly limited, but a methanol-utilizing yeast is more preferable, and a methanol-utilizing yeast belonging to the genus Ogataea or the genus Komagataella is further preferable. Among methanol-utilizing yeasts belonging to the genus Ogataea, *Ogataea polymorpha* and *Ogataea minuta* are preferable, whereas among methanol-utilizing yeasts belonging to the genus Komagataella, *Komagataella pastoris* is preferable.

The method of introducing the recombinant expression vector of the present invention into yeast is not particularly limited, as long as it is a method capable of introducing DNA into yeast. Examples of such an introduction method may include known methods including transfection methods such as a calcium phosphate method, an electroporation method, a lipofection method, a DEAE dextran method, a lithium acetate method, a spheroplast method or a protoplast method, and a microinjection method. These known methods can be used without limitation. In addition, a chromosome substitution- or insertion-type yeast transformation method of using a DNA sequence homologous to any given region in a vector such as a YIp vector or in a chromosome can also be used.

The term "transformed yeast" is used in the present invention to mean a yeast into which the recombinant vector of the present invention has been introduced. The transformed yeast of the present invention can be selectively obtained by using, as an indicator, the phenotype of an auxotrophic complementary gene or a drug resistance gene contained in the recombinant vector.

Whether or not the recombinant expression vector of the present invention has been incorporated into yeast can be confirmed by performing PCR, Southern hybridization Northern hybridization, etc. For example, DNA is prepared from the transformed yeast, DNA-specific primers are then designed, and PCR is then carried out using the primers. Thereafter, the amplification product is subjected to electrophoresis (agarose gel, polyacrylamide gel, or capillary), and the resultant is then stained with ethidium bromide, SYBR Green liquid, etc. to detect the amplification product, so that the transformation can be confirmed. Otherwise, PCR is carried out by using primers that have previously labeled with fluorescent dye, etc., so that the amplification product can be detected.

The medium for culturing the transformed yeast is not particularly limited, as long as it is a medium supplemented with a nutrient source that is utilized by yeast. As such a nutrient source, there can be used an ordinary medium that is appropriately prepared by mixing and blending: carbon sources including sugars such as glucose, sucrose or maltose, organic acids such as lactic acid, acetic acid, citric acid or propionic acid, alcohols such as methanol, ethanol or glycerol, hydrocarbons such as paraffin, oils such as soybean oil or rapeseed oil, or mixtures thereof; nitrogen sources such as ammonium sulfate, ammonium phosphate, urea, yeast extract, meat extract, peptone, or corn steep liquor; and further, other nutrient sources such as inorganic salts or vitamins. In particular, it is preferable to use glycerol or methanol as a carbon source. Moreover, as a culture method, yeast can be cultured according to any one of a batch culture, a continuous culture, and a dome type culture.

Culture can be carried out under ordinary conditions. For example, the yeast can be aerobically cultured at pH 2.5 to 10.0, preferably at pH 4.0 to 8.0, at a temperature of 10°C to 48°C, preferably 20°C to 42°C, more preferably 25°C to 37°C, for 10 hours to 10 days.

### [Protease precursor]

According to the present invention, provided is a protease precursor, in which a pro-sequence having an arginine or lysine residue at the C-terminus thereof is fused to the N-terminal side of the amino acid sequence of the *Fusarium oxysporum*-derived trypsin-like protease.

The protease precursor of the present invention is characterized in that:
(i) when the protease precursor is expressed in the culture supernatant of yeast, the protease, and a protease precursor consisting of the pro-sequence and the amino acid sequence of the protease, are secreted at an activity ratio of 1 : 1 or more in the culture supernatant of the yeast, and
(ii) the expressed protease precursor can be converted to a protease by the expressed protease.

The details of the *Fusarium oxysporum*-derived trypsin-like protease and the pro-sequence are as described above in the present description.

By culturing the above-described transformed yeast, the protease precursor of the present invention can be produced in the transformed yeast. Accordingly, the protease precursor of the present invention can be obtained according to secretory production, by which the transformed yeast of the present invention is cultured and the protease precursor of the present invention is then accumulated in the culture supernatant.

The secretory production applied in the present invention means that the transformed yeast is subjected to liquid culture, and then, a protease precursor and a protease are accumulated not inside the cell mass, but in the culture supernatant.

[Yeast culture-derived protease composition comprising *Fusarium oxysporum*-derived trypsin-like protease]

According to the present invention, provided is a yeast culture-derived protease composition comprising a *Fusarium oxysporum*-derived trypsin-like protease that is expressed in yeast. The composition of the present invention is a yeast culture-derived protease composition, which may comprise a yeast culture-derived component in some cases. In addition, the *Fusarium oxysporum*-derived trypsin-like protease of the present invention is expressed in yeast, and in some cases, the present *Fusarium oxysporum*-derived trypsin-like protease is undergone post-translational modification in the yeast.

As described above in the present description, the *Fusarium oxysporum*-derived trypsin-like protease is preferably any one of the following (a) to (c):
(a) a protease consisting of the amino acid sequence as set forth in SEQ ID NO: 67;
(b) a protease consisting of an amino acid sequence having a sequence identity of 90% or more (preferably 95% or more, and more preferably 98% or more) to the amino acid sequence as set forth in SEQ ID NO: 67, and having a trypsin-like protease activity equivalent to that of the protease consisting of the amino acid sequence as set forth in SEQ ID NO: 67; or
(c) a protease consisting of an amino acid sequence comprising a substitution, deletion and/or addition of one or several (preferably 1 to 10, more preferably 1 to 5, and further preferably 1 to 3) amino acids with respect to the amino acid sequence as set forth in SEQ ID NO: 67, and having a trypsin-like protease activity equivalent to that of the protease consisting of the amino acid sequence as set forth in SEQ ID NO: 67.

The culture supernatant or the culture obtained after the transformed yeast of the present invention has been cultured comprises a *Fusarium oxysporum*-derived trypsin-like protease and a precursor thereof (protease precursor). In the present invention, conversion of the protease precursor to a *Fusarium oxysporum*-derived trypsin-like protease is preferably carried out by subjecting the above-described culture supernatant or culture to autodigestion. Such autodigestion can be carried out by preparing a reaction solution that is under conditions suitable for the autodigestion reaction, from the above-described culture supernatant or culture, and then incubating the obtained reaction solution. The autodigestion reaction can be carried out at pH 6 to 10, more preferably at pH 7 to 9, in the presence of Mg²⁺ ions, and at a temperature of 0°C to 40°C, more preferably at a temperature of 4°C to 20°C.

Isolation and purification of the *Fusarium oxysporum*-derived trypsin-like protease can be carried out by using known protein purification methods in appropriate combination with one another. For example, the transformed yeast is cultured in a suitable medium, and then, a cell mass is removed from the culture supernatant by centrifuging the culture solution or performing a filtration treatment on the culture solution. Subsequently, the obtained culture supernatant can be subjected to autodigestion, as desired. Thereafter, the reaction solution obtained after completion of the autodigestion is subjected to means such as salting out (ammonium sulfate precipitation, sodium phosphate precipitation, etc.), solvent precipitation (a protein fractional precipitation method using acetone, ethanol, or the like), dialysis, gel filtration chromatography, ion exchange chromatography, hydrophobic chromatography, affinity chromatography, reverse phase chromatography, and ultrafiltration, so that a *Fusarium oxysporum*-derived trypsin-like protease can be recovered.

The *Fusarium oxysporum*-derived trypsin-like protease which was recovered as described above can be directly used as is, but it may also be used after various types of formulation treatments have been performed thereon.

The protease composition of the present invention can be used as a proteolytic enzyme. The pH and temperature applied in the enzyme reaction may be set in a range in which the trypsin-like protease contained in the protease composition of the present invention can exhibit a trypsin activity. The enzyme reaction is carried out under conditions of: a pH value of, for example, pH 6 to 12, and more preferably pH 7 to 10; and a temperature of 4°C to 40°C, and more preferably a temperature of 20°C to 37°C.

The intended use of the yeast culture-derived protease composition of the present invention is not particularly limited. For example, when a nucleic acid such as DNA is prepared from tissues or cells, the culture-derived protease composition can be utilized as a reagent for pre-treating a sample (a research reagent, etc.). Moreover, the yeast culture-derived protease composition of the present invention can be used in detergents (detergents for medical devices, detergents for dishwashers, detergents for washing, etc.), feed processing, food processing (fish oil processing, meat processing, etc.), textile processing, wool processing, leather processing, contact lens cleaning, pipe cleaning, medicaments, etc. Furthermore, the yeast culture-derived protease composition may also be mixed into a bathwater additive or a hair removal agent.

### Examples

Hereinafter, the present invention will be described in detail in the following examples. However, these examples are not intended to limit the scope of the present invention. Besides, detailed operation procedures and the like regarding the recombinant DNA technique applied in the following examples are described in the following publications: Molecular Cloning 2nd Edition (Cold Spring Harbor Laboratory Press, 1989), and Current Protocolsin Molecular Biology (Greene Publishing Associates and Wiley-Interscience).

Moreover, in the following examples, a plasmid used in transformation of yeast was prepared by introducing a constructed vector into *E. coli* DH5α competent cells (manufactured by Takara Bio, Inc.) and then culturing the obtained transformant to amplify it. Preparation of a plasmid from a plasmid-retaining strain was carried out by using QIAprep spin miniprep kit (manufactured by QIAGEN).

The AOX1 promoter (SEQ ID NO: 2), the AOX1 terminator (SEQ ID NO: 3), and the HIS4 gene (SEQ ID NO: 4), which were utilized in construction of the vector, were prepared by PCR using, as a template, a chromosomal DNA mixture of *Komagataella pastoris* ATCC76273 strain (the nucleotide sequences of which are described in EMBL (the European Molecular Biology Laboratory) ACCESSION Nos. FR839628 to FR839631).

With regard to a Mating Factor α prepro signal sequence (MF sequence)-added wild-type protease precursor gene (SEQ ID NO: 5), which was utilized in construction of the vector, synthetic DNA was prepared based on published sequence information.

Using Prime STAR HS DNA Polymerase (manufactured by Takara Bio, Inc.), etc., PCR was carried out according to the methods and reaction conditions described in the instruction manual included with the kit. Chromosomal DNA was prepared from the *Komagataella pastoris* ATCC76273 strain, using Dr. GenTLE™ (manufactured by Takara Bio, Inc.), etc., under the conditions described in the kit.

### Comparative Example 1: Construction of secretory expression vector for wild-type protease precursor

A gene fragment (SEQ ID NO: 6) having the multicloning site of HindIII-BamHI-BglII-XbaI-EcoRI was totally synthesized, and it was then inserted between the HindIII-EcoRI sites of pUC19 (manufactured by Takara Bio, Inc., Code No. 3219) to construct pUC-1.

Moreover, a nucleic acid fragment, in which BamHI recognition sequences were added to both sides of the AOX1 promoter, was prepared by PCR using primer 1 (SEQ ID NO: 7) and 2 (SEQ ID NO: 8). The nucleic acid fragment was treated with BamHI, and was then inserted into the BamHI site of pUC-1 to construct pUC-Paox.

Next, a nucleic acid fragment, in which XbaI recognition sequences were added to both sides of the AOX1 terminator, was prepared by PCR using primer 3 (SEQ ID NO: 9) and 4 (SEQ ID NO: 10). The nucleic acid fragment was treated with XbaI, and was then inserted into the XbaI site of pUC-Paox to construct pUC-PaoxTaox.

Next, a nucleic acid fragment, in which EcoRI recognition sequences were added to both sides of the HIS4 gene, was prepared by PCR using primer 5 (SEQ ID NO: 11) and 6 (SEQ ID NO: 12). The nucleic acid fragment was treated with EcoRI, and was then inserted into the EcoRI site of pUC-PaoxTaox to construct pUC-PaoxTaoxHIS4.

Next, a nucleic acid fragment, in which BglII recognition sequences were added to both sides of the MF sequence-added protease precursor gene, was prepared by PCR using primer 7 (SEQ ID NO: 13) and 8 (SEQ ID NO: 14). The nucleic acid fragment was treated with BglII, and was then inserted into the BglII site of pUC-PaoxTaoxHIS4 to construct pUC-PaoxTPTaoxHIS4. This pUC-PaoxTPTaoxHIS4 is designed, such that the wild-type protease precursor gene is secreted and expressed under the control of the AOX1 promoter.

### Comparative Example 2: Obtainment of transformed yeast

Using the wild-type protease precursor expression vector constructed in Comparative Example 1, *Komagataella pastoris* was transformed as follows.

A histidine auxotrophic strain derived from the *Komagataella pastoris* ATCC76273 strain was inoculated into 3 ml of YPD medium (1% yeast extract bacto (manufactured by Difco), 2% polypeptone (manufactured by NIHON PHARMACEUTICAL CO., LTD.), and 2% glucose), and the obtained mixture was then subjected to shaking culture overnight at 30°C, so as to obtain a pre-culture solution. The obtained pre-culture solution (500 µl) was inoculated into 50 ml of YPD medium, and the obtained mixture was then subjected to shaking culture until OD600 became 1 to 1.5. Thereafter, the cells were harvested (3000 x g, 10 minutes, 20°C), and were then re-suspended in 10 ml of 50 mM potassium phosphate buffer (pH 7.5) containing 250 µl of 1 M DTT (final concentration: 25 mM).

This suspension was incubated at 30°C for 15 minutes, and the cells were harvested (3000 x g, 10 minutes, 20°C), and were then washed with 50 ml of STM buffer (270 mM sucrose, 10 mM Tris-HCl, and 1 mM magnesium chloride; pH 7.5) that had previously been cooled on ice. The cells were harvested from the washing solution (3000 x g, 10 minutes, 4°C), and were then washed with 25 ml of STM buffer again. Thereafter, the cells were harvested from the resulting solution (3000 x g, 10 minutes, 4°C). Finally, the cells were suspended in 250 µl of STM buffer cooled on ice, to obtain a competent cell solution.

*Escherichia coli* was transformed using the wild-type protease precursor expression vector pUC-PaoxTPTaoxHIS4 constructed in Comparative Example 1, and the obtained transformant was then cultured in 2YT medium containing 2 ml of ampicillin (1.6% tryptone bacto (manufactured by Difco), 1% yeast extract bacto (manufactured by Difco), 0.5% sodium chloride, and 0.01% ampicillin sodium (manufactured by Wako Pure Chemical Industries, Ltd.)). Thereafter, using QIAprep spin miniprep kit (manufactured by QIAGEN), pUC-PaoxTPTaoxHIS4 was obtained from the obtained cell mass. The present plasmid was treated with SalI, so as to prepare a linear vector cleaved with the SalI recognition sequences in the HIS4 gene.

The aforementioned competent cell solution (60 µl) was mixed with the linearized pUC-PaoxTPTaoxHIS4 solution (1 µl), and the mixed solution was then transferred into a cuvette for electroporation (disposable cuvette electrode; electrode interval: 2 mm (manufactured by BM Equipment Co., Ltd.)), followed by subjecting it to 7.5 kV/cm, 25 µF, and 200 Ω. After that, the cell mass was suspended in 1 ml of YPD medium, and the suspension was then left at rest at 30°C for 1 hour. After the suspension had been left at rest for 1 hour, the cells were harvested (3000 x g, 5 minutes, 20°C), and were then suspended in 1 ml of YNB medium (0.67% yeast nitrogen base Without Amino Acid (manufactured by Difco)). Thereafter, the cells were harvested again (3000 x g, 5 minutes, 20°C). The cell mass was re-suspended in a suitable amount of YNB medium, and the obtained suspension was then applied onto a YNB-selective agar plate (0.67% yeast nitrogen base Without Amino Acid (manufactured by Difco), 1.5% agarose, and 2% glucose). Thereafter, a strain growing in a static culture at 30°C for 3 days was selected, so that a wild-type protease precursor expressing yeast was obtained.

### Comparative Example 3: Culture of transformed yeast

The wild-type protease precursor expressing yeast obtained in Comparative Example 2 was inoculated into 2 ml of BMGY medium (1% yeast extract bacto (manufactured by Difco), 2% polypeptone (manufactured by NIHON PHARMACEUTICAL CO., LTD.), 0.34% yeast nitrogen base Without Amino Acid and Ammonium Sulfate (manufactured by Difco), 1% Ammonium Sulfate, 0.4 mg/l Biotin, 100 mM potassium phosphate (pH 6.0), 1% glycerol, and 1% Methanol), and the obtained mixture was then subjected to shaking culture at 30°C for 48 hours. Thereafter, the obtained mixture was centrifuged (12000 rpm, 5 minutes, 4°C) to recover a culture supernatant.

### Comparative Example 4: SPS-PAGE of culture supernatant

The culture supernatant obtained in Comparative Example 3 was subjected to SDS-PAGE analysis.

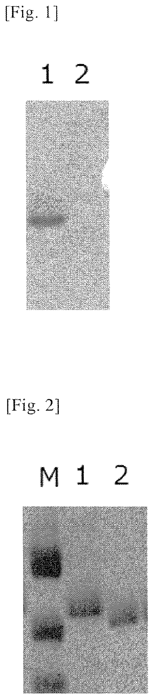

The culture supernatant (12 µl) was mixed with 3 µl of a 5 x sample buffer (0.25 M Tris-HCl (pH 6.8), 50% Glycerol, 6.7% SDS, and 0.01% BPB), followed by treating at 95°C for 8 minutes. The present sample, together with a molecular weight marker (Precision Plus Protein'™ Dual Color Standards, manufactured by Bio-Rad), was subjected to SDS-PAGE electrophoresis using e-PAGEL gel (E-R15L, manufactured by ATTO). After completion of the electrophoresis, the gel was washed with water for 15 minutes, and was then stained with a staining solution (Bio-Safe CBB G-250 Stain; manufactured by Bio-Rad) for 30 minutes. Thereafter, the resultant was decolorized with water. As a result, a band was found in a region corresponding to a molecular weight that was estimated from the amino acid sequence of the protease precursor (Fig. 1, Lane 1).

### Comparative Example 5: Conversion of protease precursor by autodigestion

The protease precursor existing in the culture supernatant was converted to an active form by autodigestion as follows.

The culture supernatant (500 µl) was substituted with an activity measuring buffer (10 mM Tris-HCl (pH 8.0) and 10 mM MgCl2), by using Amicon Ultracel-3K (manufactured by Merck Millipore). Thereafter, using the same buffer as mentioned above, the liquid amount was adjusted to 500 µl, followed by incubation at 15°C for 24 to 48 hours.

### Comparative Example 6: Measurement of protease activity

The activity of protease contained in each sample was measured by the following method.

To 990 µl of the activity measuring buffer, 10 µl of a DMSO solution of 2.5 mM N-Benzoyl-DL-arginine-p-nitroanilide HCl (manufactured by Nacalai) was added, and 1 to 10 µl of the sample was then added thereto. Then, the absorbance at 410 nm was measured at the initial stage. Thereafter, the mixture was incubated at 37°C for 1 hour, and the absorbance at 410 nm was then measured after completion of the reaction. From a change in the absorbance at 410 nm before and after the reaction, the amount of p-nitroanilline released was calculated, and the protease activity was then calculated. It is to be noted that the amount of enzyme necessary for generating 1 µmol p-nitroanilline for 1 minute was defined as 1 unit.

According to the above-described method, the protease activity in the culture supernatant obtained in Comparative Example 3, and the protease activity in the autodigested solution obtained in Comparative Example 5 were measured. However, the protease activity could not be detected (Table 1).

### Comparative Example 7: Construction of protease expression vector

A protease gene was prepared by PCR, using a synthetic gene of the MF sequence-added wild-type protease precursor gene as a template. PCR was carried out using primer 7 and primer 9 (SEQ ID NO: 15), and also using primer 10 (SEQ ID NO: 16) and primer 8, and the amplification fragments obtained from individual PCR reactions were then mixed with each other. Thereafter, the PCR was carried out using primer 7 and primer 8, so as to prepare an MF sequence-added protease gene.

The thus prepared protease gene was treated with BglII, and was then inserted into the BglII site of the pUC-PaoxTaoxHIS4 prepared in Comparative Example 1, so as to construct an MF sequence-added protease gene expression vector.

### Comparative Example 8: Secretory production of protease

Using the vector constructed in Comparative Example 7, the treatments of Comparative Examples 2 to 6 were carried out, and the secretory production of a protease in Pichia yeast was then evaluated. As a result, a band of protease was not observed on PAGE, although a small level of protease activity was found in the culture supernatant (Fig. 1, Lane 2; Table 1). The present results show that it is difficult to secrete protease at a high level in Pichia yeast.

### Comparative Example 9: Construction of mutant protease precursor expression vector

Using a synthetic gene of the MF sequence-added wild-type protease precursor gene as a template, a mutant protease precursor gene having a pig trypsin-derived pro-sequence was prepared by PCR. The PCR was carried out using primer 7 and primer 11 (SEQ ID NO: 17), and also using primer 12 (SEQ ID NO: 18) and primer 8, and the amplification fragments obtained from individual PCR reactions were then mixed with each other. Thereafter, the PCR was carried out using primer 7 and primer 8, so as to prepare an MF sequence-added mutant protease precursor gene.

The thus prepared protease gene was treated with BglII, and was then inserted into the BglII site of the pUC-PaoxTaoxHIS4 prepared in Comparative Example 1, so as to construct an MF sequence-added mutant protease precursor gene expression vector.

### Comparative Example 10: Secretory production of mutant protease precursor

Using the vector constructed in Comparative Example 9, the treatments of Comparative Examples 2 to 6 were carried out, and the secretory production of a mutant protease precursor in Pichia yeast was then evaluated. As a result, protease activity was not found in the culture supernatant (Table 1), and on PAGE, a band having a size that was different from the size estimated from the amino acid sequence was observed. The present results show that even using the pro-sequence of a pig-derive trypsin, regarding which convention of a precursor to an active form by autodigestion had been reported, it does not function well in the present protease.

**[Table 1]**

| Table 1 (Comp. Ex.1) | | | | |
|---|---|---|---|---|
| | | Protease activity (U/ml) | | |
| Secretion signal | Pro-sequence | Culture supernatant | After autodigestion | Precursor band |
| MF | APQEIPN | 0 | 0 | Yes |
| MF | - | 0.005 | 0.005 | No |
| MF | FPVDDDDK | 0 | 0 | Yes, abnormal size |

### Example 1: Construction 1 of mutant protease precursor expression vectors

Using a synthetic gene of the MF sequence-added wild-type protease precursor gene as a template, an N7NR mutant precursor gene was prepared by PCR. The PCR was carried out using primer 7 and primer 13 (SEQ ID NO: 19), and also using primer 14 (SEQ ID NO: 20) and primer 8, and the amplification fragments obtained from individual PCR reactions were then mixed with each other. Thereafter, the PCR was carried out using primer 7 and primer 8, so as to prepare an MF sequence-added N7NR mutant precursor gene.

The thus prepared mutant protease precursor gene was treated with BglII, and was then inserted into the BglII site of the pUC-PaoxTaoxHIS4 prepared in Comparative Example 1, so as to construct various types of mutant protease precursor gene expression vectors, to which an MF sequence was added.

This mutant precursor has a mutation, in which an R residue is inserted between residues corresponding to N7 and 18 of a wild-type precursor.

### Example 2: Construction 2 of mutant protease precursor expression vectors

Using a synthetic gene of the MF sequence-added wild-type protease precursor gene as a template, an N7NK mutant precursor gene was prepared by PCR. The PCR was carried out using primer 7 and primer 15 (SEQ ID NO: 21), and also using primer 16 (SEQ ID NO: 22) and primer 8, and the amplification fragments obtained from individual PCR reactions were then mixed with each other. Thereafter, the PCR was carried out using primer 7 and primer 8, so as to prepare an MF sequence-added N24NK mutant precursor gene.

The thus prepared mutant protease precursor gene was treated with BglII, and was then inserted into the BglII site of the pUC-PaoxTaoxHIS4 prepared in Comparative Example 1, so as to construct various types of mutant protease precursor gene expression vectors, to which an MF sequence was added.

This mutant precursor has a mutation, in which a K residue is inserted between residues corresponding to N7 and 18 of a wild-type precursor.

### Example 3: Construction 3 of mutant protease precursor expression vectors

The N7NR mutant precursor gene expression vector constructed in Example 1 was used as a template, and PCR was carried out by using the combinations of individual primers (1st PCR 1, 1st PCR-2) as shown in the following Table 2. Thereafter, a mixture of the obtained fragments was used as a template, and PCR was carried out using primer 7 and primer 8, so as to prepare various types of mutant protease precursor genes, to which an MF sequence was added.

The thus prepared mutant protease precursor genes were treated with BglII, and were then inserted into the BglII site of the pUC-PaoxTaoxHIS4 prepared in Comparative Example 1, so as to construct various types of mutant protease precursor gene expression vectors, to which an MF sequence was added.

These mutant precursors have a mutation, in which an R residue is inserted between residues corresponding to N7 and 18 of a wild-type precursor and also, a residue corresponding to N7 is substituted with another amino acid residue.

**[Table 2]**

| Table 2 | | | | |
|---|---|---|---|---|
| Mutant name | 1st PCR-1 | | 1st PCR-2 | |
| N7GR | Primer 7 | Primer 17 (SEQ ID NO: 23) | Primer 18 (SEQ ID NO: 24) | Primer 8 |
| N7DR | Primer 7 | Primer 19 (SEQ ID NO: 25) | Primer 20 (SEQ ID NO: 26) | Primer 8 |
| N7SR | Primer 7 | Primer 21 (SEQ ID NO: 27) | Primer 22 (SEQ ID NO: 28) | Primer 8 |
| N7AR | Primer 7 | Primer 23 (SEQ ID NO: 29) | Primer 24 (SEQ ID NO: 30) | Primer 8 |

### Example 4: Construction 4 of mutant protease precursor expression vectors

The N7NK mutant precursor gene expression vector constructed in Example 2 was used as a template, and PCR was carried out by using the combinations of individual primers (1st PCR 1, 1st PCR-2) as shown in the following Table 3. Thereafter, a mixture of the obtained fragments was used as a template, and PCR was carried out using primer 7 and primer 8, so as to prepare various types of mutant protease precursor genes, to which an MF sequence was added.

The thus prepared mutant protease precursor genes were treated with BglII, and were then inserted into the BglII site of the pUC-PaoxTaoxHIS4 prepared in Comparative Example 1, so as to construct various types of mutant protease precursor gene expression vectors, to which an MF sequence was added.

Some of these mutant precursors have a mutation, in which a K residue is inserted between residues corresponding to N7 and 18 of a wild-type precursor and also, a residue corresponding to N7 is substituted with another amino acid residue. In addition, some others of these mutant precursors have a mutation, in which a K residue is inserted between residues corresponding to N7 and 18 of a wild-type precursor and the length of the pro-sequence is different.

**[Table 3]**

| Table 3 | | | | |
|---|---|---|---|---|
| Mutant name | 1st PCR-1 | | 1st PCR-2 | |
| N7SK | Primer 7 | Primer 25 (SEQ ID NO: 31) | Primer 26 (SEQ ID NO: 32) | Primer 8 |
| N7GK | Primer 7 | Primer 27 (SEQ ID NO: 33) | Primer 28 (SEQ ID NO: 34) | Primer 8 |
| N7TK | Primer 7 | Primer 29 (SEQ ID NO: 35) | Primer 30 (SEQ ID NO: 36) | Primer 8 |
| N7EK | Primer 7 | Primer 31 (SEQ ID NO: 37) | Primer 32 (SEQ ID NO: 38) | Primer 8 |
| N7VK | Primer 7 | Primer 33 (SEQ ID NO: 39) | Primer 34 (SEQ ID NO: 40) | Primer 8 |
| N7HK | Primer 7 | Primer 35 (SEQ ID NO: 41) | Primer 36 (SEQ ID NO: 42) | Primer 8 |
| N7AK | Primer 7 | Primer 37 (SEQ ID NO: 43) | Primer 38 (SEQ ID NO: 44) | Primer 8 |
| N7YK | Primer 7 | Primer 39 (SEQ ID NO: 45) | Primer 40 (SEQ ID NO: 46) | Primer 8 |
| N7IK | Primer 7 | Primer 41 (SEQ ID NO: 47) | Primer 42 (SEQ ID NO: 48) | Primer 8 |
| N7QK | Primer 7 | Primer 43 (SEQ ID NO: 49) | Primer 44 (SEQ ID NO: 50) | Primer 8 |
| N7MK | Primer 7 | Primer 45 (SEQ ID NO: 51) | Primer 46 (SEQ ID NO: 52) | Primer 8 |
| 2+N7NK | Primer 7 | Primer 47 (SEQ ID NO: 53) | Primer 48 (SEQ ID NO: 54) | Primer 8 |
| -1N7NK | Primer 7 | Primer 49 (SEQ ID NO: 55) | Primer 50 (SEQ ID NO: 56) | Primer 8 |
| -2N7NK | Primer 7 | Primer 51 (SEQ ID NO: 57) | Primer 52 (SEQ ID NO: 58) | Primer 8 |
| -3N7NK | Primer 7 | Primer 53 (SEQ ID NO: 59) | Primer 54 (SEQ ID NO: 60) | Primer 8 |
| -4N7NK | Primer 7 | Primer 55 (SEQ ID NO: 61) | Primer 56 (SEQ ID NO: 62) | Primer 8 |
| -5N7NK | Primer 7 | Primer 57 (SEQ ID NO: 63) | Primer 58 (SEQ ID NO: 64) | Primer 8 |
| -6N7NK | Primer 7 | Primer 59 (SEQ ID NO: 65) | Primer 60 (SEQ ID NO: 66) | Primer 8 |

### Example 5: Secretory production 1 of mutant protease precursors

Using various types of mutant protease precursor gene expression vectors obtained in Examples 1 and 3, the treatments described in Comparative Examples 2 to 6 were carried out, and the secretory production of mutant protease precursors in Pichia yeast was then evaluated. As a result, a protease activity was found in the culture supernatant (Table 4), and a precursor band was also detected on PAGE. Moreover, by performing an autodigestion treatment, the protease activity was significantly improved (Table 4).

These results show that the secretory production of protease can be efficiently carried out by using a mutant protease precursor having a modified pro-sequence.

**[Table 4]**

| Table 4 | | | | | |
|---|---|---|---|---|---|
| | | Protease activity (U/ml) | | Precursor/active form ratio | |
| Secretion signal | Pro-sequence | Culture supernatant | After autodigestion | | Precursor band |
| MF | APQEIPNR | 0.045 | 0.182 | 3.02 | Yes |
| MF | APQEIPGR | 0.016 | 0.261 | 15.00 | Yes |
| MF | APQEIPDR | 0.019 | 0.245 | 11.86 | Yes |
| MF | APQEIPSR | 0.031 | 0.170 | 4.50 | Yes |
| MF | APQEIPAR | 0.027 | 0.116 | 3.32 | Yes |

### Example 6: Secretory production 2 of mutant protease precursors

Using various types of mutant protease precursor gene expression vectors obtained in Examples 2 and 4, the treatments described in Comparative Examples 2 to 6 were carried out, and the secretory production of mutant protease precursors in Pichia yeast was then evaluated. As a result, a protease activity was found in the culture supernatant (Table 5), and a precursor band was also detected on PAGE. Moreover, by performing an autodigestion treatment, the protease activity was significantly improved (Table 5). As a result of the SDS-PAGE analysis of the sample before and after the autodigestion treatment, it was found that a protease precursor band disappeared due to the treatment, and was converted to a protease band (Fig. 2).

These results show that the secretory production of protease can be efficiently carried out by using a mutant protease precursor having a modified pro-sequence.

**[Table 5]**

| Table 5 | | | | | |
|---|---|---|---|---|---|
| | | Protease activity (U/ml) | | Precursor/active form ratio | |
| Secretion signal | Pro-sequence | Culture supernatant | After autodigestion | | Precursor band |
| MF | APQEIPNK | 0.004 | 0.557 | 152.8 | Yes |
| MF | APQEIPSK | 0.004 | 0.490 | 134.3 | Yes |
| MF | APQEIPGK | 0.004 | 0.455 | 124.5 | Yes |
| MF | APQEIPTK | 0.004 | 0.396 | 94.0 | Yes |
| MF | APQEIPEK | 0.002 | 0.362 | 152.5 | Yes |
| MF | APQEIPVK | 0.004 | 0.339 | 88.0 | Yes |
| MF | APQEIPHK | 0.013 | 0.338 | 24.7 | Yes |
| MF | APQEIPAK | 0.002 | 0.261 | 129.9 | Yes |
| MF | APQEIPYK | 0.048 | 0.160 | 2.3 | Yes |
| MF | APQEIPIK | 0.004 | 0.150 | 36.7 | Yes |
| MF | APQEIPQK | 0.024 | 0.148 | 5.2 | Yes |
| MF | APQEIPMK | 0.024 | 0.120 | 4.0 | Yes |
| MF | AAAPQEIPNK | 0.006 | 0.485 | 75.4 | Yes |
| MF | PQEIPNK | 0.005 | 0.485 | 98.1 | Yes |
| MF | QEIPNK | 0.007 | 0.502 | 73.9 | Yes |
| MF | EIPNK | 0.011 | 0.539 | 50.3 | Yes |
| MF | IPNK | 0.024 | 0.302 | 11.8 | Yes |
| MF | PNK | 0.025 | 0.349 | 13.1 | Yes |
| MF | NK | 0.040 | 0.095 | 1.4 | No |

### Example 7: Construction 5 of mutant protease precursor expression vectors

The N7NK mutant precursor gene expression vector constructed in Example 2 was used as a template, and PCR was carried out by using the combinations of individual primers (1st PCR 1, 1st PCR-2) as shown in the following Table 6. Thereafter, a mixture of the obtained fragments was used as a template, and PCR was carried out using primer 7 and primer 8, so as to prepare various types of mutant protease precursor genes, to which an MF sequence was added.

The thus prepared mutant protease precursor genes were treated with BglII, and were then inserted into the BglII site of the pUC-PaoxTaoxHIS4 prepared in Comparative Example 1, so as to construct various types of mutant protease precursor gene expression vectors, to which an MF sequence was added.

**[Table 6]**

| Table 6 | | | | |
|---|---|---|---|---|
| Mutant name | 1st PCR-1 | | 1st PCR-2 | |
| A1G-N7NK | Primer 7 | Primer 61 (SEQ ID NO: 68) | Primer 62 (SEQ ID NO: 69) | Primer 8 |
| P2A-N7NK | Primer 7 | Primer 63 (SEQ ID NO: 70) | Primer 64 (SEQ ID NO: 71) | Primer 8 |
| Q3A-N7NK | Primer 7 | Primer 65 (SEQ ID NO: 72) | Primer 66 (SEQ ID NO: 73) | Primer 8 |
| E4A-N7NK | Primer 7 | Primer 67 (SEQ ID NO: 74) | Primer 68 (SEQ ID NO: 75) | Primer 8 |
| 15A-N7NK | Primer 7 | Primer 69 (SEQ ID NO: 76) | Primer 70 (SEQ ID NO: 77) | Primer 8 |

Some of these mutant precursors have a mutation, in which a K residue is inserted between residues corresponding to N7 and 18 of a wild-type precursor and also, individual amino acid residues G1 to I5 are substituted with other amino acid residues.

### Example 8: Secretory production 3 of mutant protease precursors

Using various types of mutant protease precursor gene expression vectors obtained in Example 7, the treatments described in Comparative Examples 2 to 6 were carried out, and the secretory production of mutant protease precursors in Pichia yeast was then evaluated. As a result, a protease activity was found in the culture supernatant (Table 7), and a precursor band was also detected on PAGE. Moreover, by performing an autodigestion treatment, the protease activity was significantly improved (Table 7).

**[Table 7]**

| Table 7 | | | | | |
|---|---|---|---|---|---|
| | | Protease activity (U/ml) | | Precursor/active form ratio | |
| Secretion signal | Pro-sequence | Culture supernatant | After autodigestion | | Precursor band |
| MF | APQEIPNK | 0.004 | 0.557 | 152.8 | Yes |
| MF | GPQEIPNK | 0.004 | 0.493 | 117.3 | Yes |
| MF | AAQEIPNK | 0.003 | 0.481 | 138.7 | Yes |
| MF | APAEIPNK | 0.003 | 0.480 | 138.5 | Yes |
| MF | APQAIPNK | 0.023 | 0.286 | 11.6 | Yes |
| MF | APQEAPNK | 0.011 | 0.358 | 33.1 | Yes |

These results show that the secretory production of protease can be efficiently carried out by using a mutant protease precursor having a modified pro-sequence.

### Example 9: Construction 6 of mutant protease precursor expression vectors

The N7EK mutant precursor gene expression vector constructed in Example 4 was used as a template, and PCR was carried out by using the combinations of individual primers (1st PCR 1, 1st PCR-2) as shown in the following Table 8. Thereafter, a mixture of the obtained fragments was used as a template, and PCR was carried out using primer 7 and primer 8, so as to prepare various types of mutant protease precursor genes, to which an MF sequence was added.

The thus prepared mutant protease precursor genes were treated with BglII, and were then inserted into the BglII site of the pUC-PaoxTaoxHIS4 prepared in Comparative Example 1, so as to construct various types of mutant protease precursor gene expression vectors, to which an MF sequence was added.

**[Table 8]**

| Table 8 | | | | |
|---|---|---|---|---|
| Mutant name | 1st PCR-1 | | 1st PCR-2 | |
| N7EK-R111A | Primer 7 | Primer 71 (SEQ ID NO: 78) | Primer 72 (SEQ ID NO: 79) | Primer 8 |
| N7EK-R111F | Primer 7 | Primer 73 (SEQ ID NO: 80) | Primer 74 (SEQ ID NO: 81) | Primer 8 |

These mutant precursors have a mutation, in which a K residue is inserted between residues corresponding to N7 and 18 of a wild-type precursor and also, a residue corresponding to N7 is substituted with an E residue. Moreover, these mutant precursors also have a mutation, in which R111 that is one site capable of taking place autolysis inside of the active form is substituted with another amino acid residue.

### Example 10: Secretory production 4 of mutant protease precursors

Using various types of mutant protease precursor gene expression vectors obtained in Example 9, the treatments described in Comparative Examples 2 to 6 were carried out, and the secretory production of mutant protease precursors in Pichia yeast was then evaluated. As a result, a protease activity was found in the culture supernatant (Table 9), and a precursor band was also detected on PAGE. Moreover, by performing an autodigestion treatment, the protease activity was significantly improved (Table 9).

**[Table 9]**

| Table 9 | | | | | |
|---|---|---|---|---|---|
| | | Protease activity (U/ml) | | Precursor/active form ratio | |
| pro-sequence | Mutated site | Culture supernatant | After autodigestion | | Precursor band |
| APQEIPEK | R111A | 0.002 | 0.422 | 191.5 | Yes |
| APQEIPEK | R111F | 0.001 | 0.449 | 309.4 | Yes |
| APQEIPEK | - | 0.002 | 0.362 | 152.5 | Yes |

These results show that, by modifying the R111 residue, the ratio of precursor/active form can be improved, and the secretory production of protease can be more efficiently carried out.

### Example 11: Construction of mutant protease precursor expression vector under GAP promoter

The N7EK-R111A mutant precursor gene expression vector constructed in Example 9 was treated with BamHI, and was then subjected to agarose gel electrophoresis, and the above-described vector region excluding the AOX1 promoter was purified. Subsequently, a nucleic acid fragment, in which BamHI recognition sequences were added to both sides of the GAP promoter (SEQ ID NO: 82), was prepared by PCR using primer 75 (SEQ ID NO: 83) and primer 76 (SEQ ID NO: 84), and was then treated with BamHI. Thereafter, the resulting nucleic acid fragment was inserted into the BamHI site of the above-described vector region, so as to construct an expression vector, in which the N7EK-R111A mutant precursor gene was expressed under the GAP promoter.

### Example 12: Secretory production 4 of mutant protease precursors

Using the expression vector for expressing the N7EK-R111A mutant precursor gene under the GAP promoter which was obtained in Example 11, the treatments described in Comparative Examples 2 to 6 were carried out, and the secretory production of the mutant protease precursor in Pichia yeast was then evaluated. As a result, a protease activity was found in the culture supernatant (Table 10), and a precursor band was also detected on PAGE. Moreover, by performing an autodigestion treatment, the protease activity was significantly improved (Table 10).

**[Table 10]**

| Table 10 | | | | | |
|---|---|---|---|---|---|
| | | Protease activity (U/ml) | | Precursor/active form ratio | |
| Promoter | Mutant | Culture supernatant | After autodigestion | | Precursor band |
| AOX1 | N7EK-R111A | 0.002 | 0.422 | 191.5 | Yes |
| GAP | N7EK-R111A | 0.001 | 0.457 | 349.7 | Yes |

These results show that, by changing the promoter, the ratio of precursor/active form can be improved, and the secretory production of protease can be more efficiently carried out.

Page 1 of 13
## Claims

1. A method for producing a protease in yeast, which comprises expressing a gene encoding a fusion protein having a secretion signal sequence functioning in yeast, a pro-sequence of a *Fusarium oxysporum*-derived trypsin-like protease, into which a mutation is introduced and which has an arginine or lysine residue at the C-terminus thereof, and the amino acid sequence of the *Fusarium oxysporum*-derived trypsin-like protease, in this order from the N-terminal side to C-terminal side thereof, in yeast.

2. The method according to claim 1, wherein the protease, and a protease precursor consisting of the pro-sequence and the amino acid sequence of the protease, are secreted at an activity ratio of 1 : 1 or more in the culture supernatant of the yeast.

3. The method according to claim 1 or 2, wherein the protease precursor expressed in the culture supernatant is converted to a protease by the protease expressed in the culture supernatant.

4. The method according to any one of claims 1 to 3, wherein the *Fusarium oxysporum*-derived trypsin-like protease is any one of the following (a) to (c):
(a) a protease consisting of the amino acid sequence as set forth in SEQ ID NO: 67;
(b) a protease consisting of an amino acid sequence having a sequence identity of 90% or more to the amino acid sequence as set forth in SEQ ID NO: 67, and having a trypsin-like protease activity equivalent to that of the protease consisting of the amino acid sequence as set forth in SEQ ID NO: 67; or
(c) a protease consisting of an amino acid sequence comprising a substitution, deletion and/or addition of one or several amino acids with respect to the amino acid sequence as set forth in SEQ ID NO: 67, and having a trypsin-like protease activity equivalent to that of the protease consisting of the amino acid sequence as set forth in SEQ ID NO: 67.

5. The method according to any one of claims 1 to 4, wherein the secretion signal sequence functioning in yeast is a yeast MF sequence.

6. The method according to any one of claims 1 to 5, wherein the pro-sequence is an amino acid sequence comprising an addition, deletion or substitution of one or several amino acids with respect to the amino acid sequence shown in Ala-Pro-Gln-Glu-Ile-Pro-Asn, and the amino acid sequence has an arginine or lysine residue at the C-terminus thereof, and is cleaved at the C-terminus thereof in yeast, so that a protease is generated from the protease precursor.

7. The method according to any one of claims 1 to 6, wherein the pro-sequence is an amino acid sequence shown in any one of the following:
Ala- Pro-Gln-Glu-Ile-Pro-Xaa-Xbb,
Xcc-Ala- Pro-Gln-Glu-Ile-Pro-Xaa-Xbb,
Xdd-Xcc-Ala- Pro-Gln-Glu-Ile-Pro-Xaa-Xbb,
Pro-Gln-Glu-Ile-Pro-Xaa-Xbb,
Gln-Glu-Ile-Pro-Xaa-Xbb,
Glu-Ile-Pro-Xaa-Xbb,
Ile-Pro-Xaa-Xbb,
Pro-Xaa-Xbb, or
Xaa-Xbb,
wherein Xaa represents any given amino acid residue, Xbb represents an Arg or Lys residue,
Xcc represents any given amino acid residue, and Xdd represents any given amino acid residue.

8. A protease precursor, in which a pro-sequence having an arginine or lysine residue at the C-terminus thereof is fused to the N-terminal side of the amino acid sequence of the *Fusarium oxysporum*-derived trypsin-like protease, wherein
(i) when the protease precursor is expressed in the culture supernatant of yeast, the protease, and a protease precursor consisting of the pro-sequence and the amino acid sequence of the protease, are secreted at an activity ratio of 1 : 1 or more in the culture supernatant of the yeast, and
(ii) the expressed protease precursor can be converted to a protease by the expressed protease.

9. The protease precursor according to claim 8, wherein the *Fusarium oxysporum*-derived trypsin-like protease is any one of the following (a) to (c):
(a) a protease consisting of the amino acid sequence as set forth in SEQ ID NO: 67;
(b) a protease consisting of an amino acid sequence having a sequence identity of 90% or more to the amino acid sequence as set forth in SEQ ID NO: 67, and having a trypsin-like protease activity equivalent to that of the protease consisting of the amino acid sequence as set forth in SEQ ID NO: 67; or
(c) a protease consisting of an amino acid sequence comprising a substitution, deletion and/or addition of one or several amino acids with respect to the amino acid sequence as set forth in SEQ ID NO: 67, and having a trypsin-like protease activity equivalent to that of the protease consisting of the amino acid sequence as set forth in SEQ ID NO: 67.

10. The protease precursor according to claim 8 or 9, wherein the pro-sequence is an amino acid sequence comprising an addition, deletion or substitution of one or several amino acids with respect to the amino acid sequence shown in
Ala-Pro-Gln-Glu-Ile-Pro-Asn, and the amino acid sequence has an arginine or lysine residue at the C-terminus thereof, and is cleaved at the C-terminus thereof in yeast, so that a protease is generated from the protease precursor.

11. The protease precursor according to any one of claims 8 to 10, wherein the pro-sequence is an amino acid sequence shown in any one of the following:
Ala- Pro-Gln-Glu-Ile-Pro-Xaa-Xbb,
Xcc-Ala- Pro-Gln-Glu-Ile-Pro-Xaa-Xbb,
Xdd-Xcc-Ala- Pro-Gln-Glu-Ile-Pro-Xaa-Xbb,
Pro-Gln-Glu-Ile-Pro-Xaa-Xbb,
Gln-Glu-Ile-Pro-Xaa-Xbb,
Glu-Ile-Pro-Xaa-Xbb,
Ile-Pro-Xaa-Xbb,
Pro-Xaa-Xbb, or
Xaa-Xbb,
wherein Xaa represents any given amino acid residue, Xbb represents an Arg or Lys residue,
Xcc represents any given amino acid residue, and Xdd represents any given amino acid residue.

12. A recombinant expression vector having DNA that encodes a fusion protein having a secretion signal sequence functioning in yeast, a pro-sequence of a *Fusarium oxysporum*-derived trypsin-like protease, into which a mutation is introduced and which has an arginine or lysine residue at the C-terminus thereof, and the amino acid sequence of the *Fusarium oxysporum*-derived trypsin-like protease, in this order from the N-terminal side to C-terminal side thereof.

13. The recombinant expression vector according to claim 12, wherein the *Fusarium oxysporum*-derived trypsin-like protease is any one of the following (a) to (c):
(a) a protease consisting of the amino acid sequence as set forth in SEQ ID NO: 67;
(b) a protease consisting of an amino acid sequence having a sequence identity of 90% or more to the amino acid sequence as set forth in SEQ ID NO: 67, and having a trypsin-like protease activity equivalent to that of the protease consisting of the amino acid sequence as set forth in SEQ ID NO: 67; or
(c) a protease consisting of an amino acid sequence comprising a substitution, deletion and/or addition of one or several amino acids with respect to the amino acid sequence as set forth in SEQ ID NO: 67, and having a trypsin-like protease activity equivalent to that of the protease consisting of the amino acid sequence as set forth in SEQ ID NO: 67.

14. The recombinant expression vector according to claim 12 or 13, wherein the secretion signal sequence functioning in yeast is a yeast MF sequence.

15. The recombinant expression vector according to any one of claims 12 to 14, wherein the pro-sequence is an amino acid sequence comprising an addition, deletion or substitution of one or several amino acids with respect to the amino acid sequence shown in Ala- Pro-Gln-Glu-Ile-Pro-Asn, and the amino acid sequence is cleaved at the C-terminus thereof in yeast, so that a protease is generated from the protease precursor.

16. The recombinant expression vector according to any one of claims 12 to 15, wherein the pro-sequence is an amino acid sequence shown in any one of the following:
Ala- Pro-Gln-Glu-Ile-Pro-Xaa-Xbb,
Xcc-Ala- Pro-Gln-Glu-Ile-Pro-Xaa-Xbb,
Xdd-Xcc-Ala- Pro-Gln-Glu-Ile-Pro-Xaa-Xbb,
Pro-Gln-Glu-Ile-Pro-Xaa-Xbb,
Gln-Glu-Ile-Pro-Xaa-Xbb,
Glu-Ile-Pro-Xaa-Xbb,
Ile-Pro-Xaa-Xbb,
Pro-Xaa-Xbb, or
Xaa-Xbb,
wherein Xaa represents any given amino acid residue, Xbb represents an Arg or Lys residue,
Xcc represents any given amino acid residue, and Xdd represents any given amino acid residue.

17. A transformed yeast having the recombinant expression vector according to any one of claims 12 to 16.


SEQUENCE LISTING
<110>  Kaneka
       Nishiyama, Tozo
<120>  Trypsin-like-protease
<130>  181046A
<160>  84
<170>  PatentIn version 3.5
<210>  1
<211>  231
<212>  PRT
<213>  Fusarium oxysporum
<400>  1
Ala Pro Gln Glu Ile Pro Asn Ile Val Gly Gly Thr Ser Ala Ser Ala
1               5                   10                  15
Gly Asp Phe Pro Phe Ile Val Ser Ile Ser Arg Asn Gly Gly Pro Trp
            20                  25                  30
Cys Gly Gly Ser Leu Leu Asn Ala Asn Thr Val Leu Thr Ala Ala His
        35                  40                  45
Cys Val Ser Gly Tyr Ala Gln Ser Gly Phe Gln Ile Arg Ala Gly Ser
    50                  55                  60
Leu Ser Arg Thr Ser Gly Gly Ile Thr Ser Ser Leu Ser Ser Val Arg
65                  70                  75                  80
Val His Pro Ser Tyr Ser Gly Asn Asn Asn Asp Leu Ala Ile Leu Lys
                85                  90                  95
Leu Ser Thr Ser Ile Pro Ser Gly Gly Asn Ile Gly Tyr Ala Arg Leu
            100                 105                 110
Ala Ala Ser Gly Ser Asp Pro Val Ala Gly Ser Ser Ala Thr Val Ala
        115                 120                 125
Gly Trp Gly Ala Thr Ser Glu Gly Gly Ser Ser Thr Pro Val Asn Leu
    130                 135                 140
Leu Lys Val Thr Val Pro Ile Val Ser Arg Ala Thr Cys Arg Ala Gln
145                 150                 155                 160
Tyr Gly Thr Ser Ala Ile Thr Asn Gln Met Phe Cys Ala Gly Val Ser
                165                 170                 175
Ser Gly Gly Lys Asp Ser Cys Gln Gly Asp Ser Gly Gly Pro Ile Val
            180                 185                 190
Asp Ser Ser Asn Thr Leu Ile Gly Ala Val Ser Trp Gly Asn Gly Cys
        195                 200                 205
Ala Arg Pro Asn Tyr Ser Gly Val Tyr Ala Ser Val Gly Ala Leu Arg
    210                 215                 220
Ser Phe Ile Asp Thr Tyr Ala
225                 230
<210>  2
<211>  933
<212>  DNA
<213>  Pichia pastoris
<400>  2
aacatccaaa gacgaaaggt tgaatgaaac ctttttgcca tccgacatcc acaggtccat      60
tctcacacat aagtgccaaa cgcaacagga ggggatacac tagcagcaga ccgttgcaaa     120
cgcaggacct ccactcctct tctcctcaac acccactttt gccatcgaaa aaccagccca     180
gttattgggc ttgattggag ctcgctcatt ccaattcctt ctattaggct actaacacca     240
tgactttatt agcctgtcta tcctggcccc cctggcgagg ttcatgtttg tttatttccg     300
aatgcaacaa gctccgcatt acacccgaac atcactccag atgagggctt tctgagtgtg     360
gggtcaaata gtttcatgtt ccccaaatgg cccaaaactg acagtttaaa cgctgtcttg     420
gaacctaata tgacaaaagc gtgatctcat ccaagatgaa ctaagtttgg ttcgttgaaa     480
tgctaacggc cagttggtca aaaagaaact tccaaaagtc ggcataccgt ttgtcttgtt     540
tggtattgat tgacgaatgc tcaaaaataa tctcattaat gcttagcgca gtctctctat     600
cgcttctgaa ccccggtgca cctgtgccga aacgcaaatg gggaaacacc cgctttttgg     660
atgattatgc attgtctcca cattgtatgc ttccaagatt ctggtgggaa tactgctgat     720
agcctaacgt tcatgatcaa aatttaactg ttctaacccc tacttgacag caatatataa     780
acagaaggaa gctgccctgt cttaaacctt ttttttatca tcattattag cttactttca     840
taattgcgac tggttccaat tgacaagctt ttgattttaa cgacttttaa cgacaacttg     900
agaagatcaa aaaacaacta attattcgaa acg                                  933

Page 2 of 13

<210> 3
<211> 500
<212> DNA
<213> Pichia pastoris
<400> 3
tcaagaggat gtcagaatgc catttgcctg agagatgcag gcttcatttt tgatactttt 60
ttatttgtaa cctatatagt ataggatttt ttttgtcatt ttgtttcttc tcgtacgagc 120
ttgctcctga tcagcctatc tcgcagctga tgaatatctt gtggtagggg tttgggaaaa 180
tcattcgagt ttgatgtttt tcttggtatt tcccactcct cttcagagta cagaagatta 240
agtgagacgt tcgtttgtgc aagcttcaac gatgccaaaa gggtataata agcgtcattt 300
gcagcattgt gaagaaaact atgtggcaag ccaagcctgc gaagaatgta ttttaagttt 360
gactttgatg tattcacttg attaagccat aattctcgag tatctatgat tggaagtatg 420
ggaatggtga tacccgcatt cttcagtgtc ttgaggtctc ctatcagatt atgcccaact 480
aaagcaaccg gaggaggaga 500
<210> 4
<211> 2644
<212> DNA
<213> Pichia pastoris
<400> 4
cctgatgact gactcactga taataaaaat acggcttcag aatttctcaa gactacactc 60
actgtccgac ttcaagtatg acatttccct tgctacctgc atacgcaagt gttgcagagt 120
ttgataattc cttgagtttg gtaggaaaag ccgtgtttcc ctatgctgct gaccagctgc 180
acaacctgat caagttcact caatcgactg agcttcaagt taatgtgcaa gttgagtcat 240
ccgttacaga ggaccaattt gaggagctga tcgacaactt gctcaagttg tacaataatg 300
gtatcaatga agtgattttg gacctagatt tggcagaaag agttgtccaa aggatcccag 360
gcgctagggt tatctatagg accctggttg ataaagttgc atccttgccc gctaatgcta 420
gtatcgctgt gcctttttct tctccactgg gcgatttgaa aagtttcact aatggcggta 480
gtagaactgt ttatgctttt tctgagaccg caaagttggt agatgtgact tccactgttg 540
cttctggtat aatccccatt attgatgctc ggcaattgac tactgaatac gaactttctg 600
aagatgtcaa aaagttccct gtcagtgaaa ttttgttggc gtctttgact actgaccgcc 660
ccgatggtct attcactact ttggtggctg actcttctaa ttactcgttg ggcctggtgt 720
actcgtccaa aaagtctatt ccggaggcta taaggacaca aactggagtc taccaatctc 780
gtcgtcacgg tttgtggtat aaaggtgcta catctggagc aactcaaaag ttgctgggta 840
tcgaattgga ttgtgatgga gactgcttga aatttgtggt tgaacaaaca ggtgttggtt 900
tctgtcactt ggaacgcact tcctgttttg gccaatcaaa gggtcttaga gccatggaag 960
ccaccttgtg ggatcgtaag agcaatgctc cagaaggttc ttataccaaa cggttatttg 1020
acgacgaagt tttgttgaac gctaaaatta gggaggaagc tgatgaactt gcagaagcta 1080
aatccaagga agatatagcc tgggaatgtg ctgacttatt ttattttgca ttagttagat 1140
gtgccaagta cggtgtgacg ttggacgagg tggagagaaa cctggatatg aagtccctaa 1200
aggtcactag aaggaaagga gatgccaagc caggatacac caaggaacaa cctaaagaag 1260
aatccaaacc taaagaagtc ccttctgaag gtcgtattga attgtgcaaa attgacgttt 1320
ctaaggcctc ctcacaagaa attgaagatg cccttcgtcg tcctatccag aaaacggaac 1380
agattatgga attagtcaaa ccaattgtcg acaatgttcg tcaaaatggt gacaaagccc 1440
ttttagaact aactgccaag tttgatggag tcgctttgaa gacacctgtg ttagaagctc 1500
ctttcccaga ggaacttatg caattgccag ataacgttaa gagagccatt gatctctcta 1560
tagataacgt caggaaattc catgaagctc aactaacgga gacgttgcaa gttgagactt 1620
gccctggtgt agtctgctct cgttttgcaa gacctattga gaaagttggc ctctatattc 1680
ctggtggaac cgcaattctg ccttccactt ccctgatgct gggtgttcct gccaaagttg 1740
ctggttgcaa agaaattgtt tttgcatctc cacctaagaa ggatggtacc cttaccccag 1800
aagtcatcta cgttgcccac aaggttggtg ctaagtgtat cgtgctagca ggaggcgccc 1860
aggcagtagc tgctatggct tacggaacag aaactgttcc taagtgtgac aaaatatttg 1920
gtccaggaaa ccagttcgtt actgctgcca agatgatggt tcaaaatgac acatcagccc 1980
tgtgtagtat tgacatgcct gctgggcctt ctgaagttct agttattgct gataaatacg 2040
ctgatccaga tttcgttgcc tcagaccttc tgtctcaagc tgaacatggt attgattccc 2100
aggtgattct gttggctgtc gatatgacag acaaggagct tgccagaatt gaagatgctg 2160
ttcacaacca agctgtgcag ttgccaaggg ttgaaattgt acgcaagtgt attgcacact 2220
ctacaaccct atcggttgca acctacgagc aggctttgga aatgtccaat cagtacgctc 2280
ctgaacactt gatcctgcaa atcgagaatg cttcttctta tgttgatcaa gtacaacacg 2340
ctggatctgt gtttgttggt gcctactctc cagagagttg tggagattac tcctccggta 2400
ccaaccacac tttgccaacg tacggatatg cccgtcaata cagcggagtt aacactgcaa 2460
ccttccagaa gttcatcact tcacaagacg taactcctga gggactgaaa catattggcc 2520
aagcagtgat ggatctggct gctgttgaag gtctagatgc tcaccgcaat gctgttaagg 2580
ttcgtatgga gaaactggga cttatttaat tatttagaga ttttaactta catttagatt 2640

Page 3 of 13
cgat 2644
<210> 5
<211> 963
<212> DNA
<213> artificial
<220>
<223> artificial
<400> 5
atgagattcc catccatctt cactgctgtt ttgttcgctg cttcttccgc tttggctgct 60
ccagttaata ctactactga ggacgagact gctcagattc cagctgaagc tgttattggt 120
tactccgact tggaaggtga cttcgacgtt gctgttttgc cattctccaa ctccactaac 180
aacggtttgt tgttcatcaa cactactatc gcttccattg ctgctaaaga agagggagtt 240
tccttggaga agagagaggc tgaggctgct ccacaagaaa ttccaaatat cgttggtggt 300
acttccgctt ccgctggtga ttttccattc atcgtttcca tctccagaaa cggtggtcct 360
tggtgtggtg gttctttgtt gaacgctaac actgttttga ctgctgctca ctgtgtttcc 420
ggttacgctc aatccggttt ccaaattaga gctggttcct tgtccagaac ttccggtggt 480
attacttcct cattgtcctc tgttagagtt cacccatcct actccggtaa caacaacgac 540
ttggctatct tgaagttgtc cacttccatt ccatccggtg gaaacattgg ttacgctaga 600
ttggctgctt ctggttctga tccagttgct ggttcttctg ctactgttgc tggatggggt 660
gctacttctg aaggtggatc ttccactcca gttaatttgt tgaaggttac tgttccaatc 720
gtttctagag ctacttgtag ggctcagtac ggaacttccg ctatcactaa ccagatgttc 780
tgtgctggtg tttcctctgg tggtaaggac tcttgtcaag gtgattctgg tggtcctatc 840
gttgattcct ccaacacttt gatcggtgct gtttcttggg gtaacggttg tgctagacca 900
aactactctg gtgtttacgc ttctgttgga gctttgagat ccttcatcga cacttacgct 960
taa 963
<210> 6
<211> 30
<212> DNA
<213> Artificial Sequence
<220>
<223> artificial
<400> 6
aagcttggat ccagatcttc tagagaattc 30
<210> 7
<211> 30
<212> DNA
<213> Artificial Sequence
<220>
<223> artificial
<400> 7
ataaggatcc aacatccaaa gacgaaaggt 30
<210> 8
<211> 30
<212> DNA
<213> Artificial Sequence
<220>
<223> artificial
<400> 8
tataggatcc cgtttcgaat aattagttgt 30
<210> 9
<211> 30
<212> DNA
<213> Artificial Sequence
<220>
<223> aartificial
<400> 9
ataatctaga tcaagaggat gtcagaatgc 30
<210> 10
<211> 30
<212> DNA
<213> Artificial Sequence
<220>
<223> artificial

Page 4 of 13
<400>  10
tatatctaga tctcctcctc cggttgcttt                                        30
<210>  11
<211>  30
<212>  DNA
<213>  Artificial Sequence
<220>
<223>  artificial
<400>  11
ataagaattc cctgatgact gactcactga                                        30
<210>  12
<211>  30
<212>  DNA
<213>  Artificial Sequence
<220>
<223>  artificial
<400>  12
tatagaattc atcgaatcta aatgtaagtt                                        30
<210>  13
<211>  30
<212>  DNA
<213>  Artificial Sequence
<220>
<223>  artificial
<400>  13
ataaagatct atgagattcc catccatctt                                        30
<210>  14
<211>  30
<212>  DNA
<213>  Artificial Sequence
<220>
<223>  artificial
<400>  14
tataagatct ttaagcgtaa gtgtcgatga                                        30
<210>  15
<211>  30
<212>  DNA
<213>  Artificial Sequence
<220>
<223>  artificial
<400>  15
caccaacgat tctcttctcc aaggaaactc                                        30
<210>  16
<211>  31
<212>  DNA
<213>  Artificial Sequence
<220>
<223>  artificial
<400>  16
ggagaagaga atcgttggtg gtacttccgc t                                      31
<210>  17
<211>  44
<212>  DNA
<213>  Artificial Sequence
<220>
<223>  artificial
<400>  17
cttgtcgtcg tcgtcaactg ggaaagcctc agcctctctc ttct                        44
<210>  18
<211>  45
<212>  DNA
<213>  Artificial Sequence
<220>

Page 5 of 13
<223>  artificial
<400>  18
ttcccagttg acgacgacga caagatcgtt ggtggtactt ccgct                    45
<210>  19
<211>  33
<212>  DNA
<213>  Artificial Sequence
<220>
<223>  artificial
<400>  19
agtaccacca acgattctat ttggaatttc ttg                                 33
<210>  20
<211>  33
<212>  DNA
<213>  Artificial Sequence
<220>
<223>  artificial
<400>  20
caagaaattc caaatagaat cgttggtggt act                                 33
<210>  21
<211>  33
<212>  DNA
<213>  Artificial Sequence
<220>
<223>  artificial
<400>  21
agtaccacca acgatcttat ttggaatttc ttg                                 33
<210>  22
<211>  33
<212>  DNA
<213>  Artificial Sequence
<220>
<223>  artificial
<400>  22
caagaaattc caaataagat cgttggtggt act                                 33
<210>  23
<211>  35
<212>  DNA
<213>  Artificial Sequence
<220>
<223>  artificial
<400>  23
taccaccaac gattctacct ggaatttctt gtgga                               35
<210>  24
<211>  35
<212>  DNA
<213>  Artificial Sequence
<220>
<223>  artificial
<400>  24
tccacaagaa attccaggta gaatcgttgg tggta                               35
<210>  25
<211>  35
<212>  DNA
<213>  Artificial Sequence
<220>
<223>  artificial
<400>  25
taccaccaac gattctgtct ggaatttctt gtgga                               35
<210>  26
<211>  35
<212>  DNA
<213>  Artificial Sequence

Page 6 of 13
<220>
<223>  artificial
<400>  26
tccacaagaa attccagaca gaatcgttgg tggta                                35
<210>  27
<211>  35
<212>  DNA
<213>  Artificial Sequence
<220>
<223>  artificial
<400>  27
taccaccaac gattctggat ggaatttctt gtgga                                35
<210>  28
<211>  35
<212>  DNA
<213>  Artificial Sequence
<220>
<223>  artificial
<400>  28
tccacaagaa attccatcca gaatcgttgg tggta                                35
<210>  29
<211>  35
<212>  DNA
<213>  Artificial Sequence
<220>
<223>  artificial
<400>  29
taccaccaac gattctagct ggaatttctt gtgga                                35
<210>  30
<211>  35
<212>  DNA
<213>  Artificial Sequence
<220>
<223>  artificial
<400>  30
tccacaagaa attccagcta gaatcgttgg tggta                                35
<210>  31
<211>  35
<212>  DNA
<213>  Artificial Sequence
<220>
<223>  artificial
<400>  31
taccaccaac gatcttggat ggaatttctt gtgga                                35
<210>  32
<211>  35
<212>  DNA
<213>  Artificial Sequence
<220>
<223>  artificial
<400>  32
tccacaagaa attccatcca agatcgttgg tggta                                35
<210>  33
<211>  35
<212>  DNA
<213>  Artificial Sequence
<220>
<223>  artificial
<400>  33
taccaccaac gatcttacct ggaatttctt gtgga                                35
<210>  34
<211>  35
<212>  DNA

Page 7 of 13
<213>  Artificial Sequence
<220>
<223>  artificial
<400>  34
tccacaagaa attccaggta agatcgttgg tggta                                35
<210>  35
<211>  35
<212>  DNA
<213>  Artificial Sequence
<220>
<223>  artificial
<400>  35
taccaccaac gatcttagtt ggaatttctt gtgga                                35
<210>  36
<211>  35
<212>  DNA
<213>  Artificial Sequence
<220>
<223>  artificial
<400>  36
tccacaagaa attccaacta agatcgttgg tggta                                35
<210>  37
<211>  35
<212>  DNA
<213>  Artificial Sequence
<220>
<223>  artificial
<400>  37
taccaccaac gatcttctct ggaatttctt gtgga                                35
<210>  38
<211>  35
<212>  DNA
<213>  Artificial Sequence
<220>
<223>  artificial
<400>  38
tccacaagaa attccagaga agatcgttgg tggta                                35
<210>  39
<211>  35
<212>  DNA
<213>  Artificial Sequence
<220>
<223>  artificial
<400>  39
taccaccaac gatcttaact ggaatttctt gtgga                                35
<210>  40
<211>  35
<212>  DNA
<213>  Artificial Sequence
<220>
<223>  artificial
<400>  40
tccacaagaa attccagtta agatcgttgg tggta                                35
<210>  41
<211>  35
<212>  DNA
<213>  Artificial Sequence
<220>
<223>  artificial
<400>  41
taccaccaac gatcttgtgt ggaatttctt gtgga                                35
<210>  42
<211>  35

Page 8 of 13
<212> DNA
<213> Artificial Sequence
<220>
<223> artificial
<400> 42
tccacaagaa attccacaca agatcgttgg tggta 35
<210> 43
<211> 35
<212> DNA
<213> Artificial Sequence
<220>
<223> artificial
<400> 43
taccaccaac gatcttagct ggaatttctt gtgga 35
<210> 44
<211> 35
<212> DNA
<213> Artificial Sequence
<220>
<223> artificial
<400> 44
tccacaagaa attccagcta agatcgttgg tggta 35
<210> 45
<211> 35
<212> DNA
<213> Artificial Sequence
<220>
<223> artificial
<400> 45
taccaccaac gatcttgtat ggaatttctt gtgga 35
<210> 46
<211> 35
<212> DNA
<213> Artificial Sequence
<220>
<223> artificial
<400> 46
tccacaagaa attccataca agatcgttgg tggta 35
<210> 47
<211> 35
<212> DNA
<213> Artificial Sequence
<220>
<223> artificial
<400> 47
taccaccaac gatcttgatt ggaatttctt gtgga 35
<210> 48
<211> 35
<212> DNA
<213> Artificial Sequence
<220>
<223> artificial
<400> 48
tccacaagaa attccaatca agatcgttgg tggta 35
<210> 49
<211> 35
<212> DNA
<213> Artificial Sequence
<220>
<223> artificial
<400> 49
taccaccaac gatcttttgt ggaatttctt gtgga 35
<210> 50

Page 9 of 13
<211>  35
<212>  DNA
<213>  Artificial Sequence
<220>
<223>  artificial
<400>  50
tccacaagaa attccacaaa agatcgttgg tggta                                35
<210>  51
<211>  35
<212>  DNA
<213>  Artificial Sequence
<220>
<223>  artificial
<400>  51
taccaccaac gatcttcatt ggaatttctt gtgga                                35
<210>  52
<211>  35
<212>  DNA
<213>  Artificial Sequence
<220>
<223>  artificial
<400>  52
tccacaagaa attccaatga agatcgttgg tggta                                35
<210>  53
<211>  40
<212>  DNA
<213>  Artificial Sequence
<220>
<223>  artificial
<400>  53
ggaatttctt gtggagcagc agcagcctca gcctctctct                           40
<210>  54
<211>  40
<212>  DNA
<213>  Artificial Sequence
<220>
<223>  artificial
<400>  54
agagagaggc tgaggctgct gctgctccac aagaaattcc                           40
<210>  55
<211>  35
<212>  DNA
<213>  Artificial Sequence
<220>
<223>  artificial
<400>  55
tggaatttct tgtggagcct cagcctctct cttct                                35
<210>  56
<211>  35
<212>  DNA
<213>  Artificial Sequence
<220>
<223>  artificial
<400>  56
agagaggctg aggctccaca agaaattcca aataa                                35
<210>  57
<211>  35
<212>  DNA
<213>  Artificial Sequence
<220>
<223>  artificial
<400>  57
atttggaatt tcttgagcct cagcctctct cttct                                35

Page 10 of 13
<210> 58
<211> 35
<212> DNA
<213> Artificial Sequence
<220>
<223> artificial
<400> 58
agagaggctg aggctcaaga aattccaaat aagat 35
<210> 59
<211> 35
<212> DNA
<213> Artificial Sequence
<220>
<223> artificial
<400> 59
cttatttgga atttcagcct cagcctctct cttct 35
<210> 60
<211> 35
<212> DNA
<213> Artificial Sequence
<220>
<223> artificial
<400> 60
agagaggctg aggctgaaat tccaaataag atcgt 35
<210> 61
<211> 35
<212> DNA
<213> Artificial Sequence
<220>
<223> artificial
<400> 61
gatcttattt ggaatagcct cagcctctct cttct 35
<210> 62
<211> 35
<212> DNA
<213> Artificial Sequence
<220>
<223> artificial
<400> 62
agagaggctg aggctattcc aaataagatc gttgg 35
<210> 63
<211> 35
<212> DNA
<213> Artificial Sequence
<220>
<223> artificial
<400> 63
aacgatctta tttggagcct cagcctctct cttct 35
<210> 64
<211> 35
<212> DNA
<213> Artificial Sequence
<220>
<223> artificial
<400> 64
agagaggctg aggctccaaa taagatcgtt ggtgg 35
<210> 65
<211> 35
<212> DNA
<213> Artificial Sequence
<220>
<223> artificial
<400> 65

Page 11 of 13
accaacgatc ttattagcct cagcctctct cttct 35
<210> 66
<211> 35
<212> DNA
<213> Artificial Sequence
<220>
<223> artificial
<400> 66
agagaggctg aggctaataa gatcgttggt ggtac 35
<210> 67
<211> 224
<212> PRT
<213> Fusarium oxysporum
<400> 67
Ile Val Gly Gly Thr Ser Ala Ser Ala Gly Asp Phe Pro Phe Ile Val
1               5                   10                  15
Ser Ile Ser Arg Asn Gly Gly Pro Trp Cys Gly Gly Ser Leu Leu Asn
            20                  25                  30
Ala Asn Thr Val Leu Thr Ala Ala His Cys Val Ser Gly Tyr Ala Gln
        35                  40                  45
Ser Gly Phe Gln Ile Arg Ala Gly Ser Leu Ser Arg Thr Ser Gly Gly
    50                  55                  60
Ile Thr Ser Ser Leu Ser Ser Val Arg Val His Pro Ser Tyr Ser Gly
65                  70                  75                  80
Asn Asn Asn Asp Leu Ala Ile Leu Lys Leu Ser Thr Ser Ile Pro Ser
                85                  90                  95
Gly Gly Asn Ile Gly Tyr Ala Arg Leu Ala Ala Ser Gly Ser Asp Pro
            100                 105                 110
Val Ala Gly Ser Ser Ala Thr Val Ala Gly Trp Gly Ala Thr Ser Glu
        115                 120                 125
Gly Gly Ser Ser Thr Pro Val Asn Leu Leu Lys Val Thr Val Pro Ile
    130                 135                 140
Val Ser Arg Ala Thr Cys Arg Ala Gln Tyr Gly Thr Ser Ala Ile Thr
145                 150                 155                 160
Asn Gln Met Phe Cys Ala Gly Val Ser Ser Gly Gly Lys Asp Ser Cys
                165                 170                 175
Gln Gly Asp Ser Gly Gly Pro Ile Val Asp Ser Ser Asn Thr Leu Ile
            180                 185                 190
Gly Ala Val Ser Trp Gly Asn Gly Cys Ala Arg Pro Asn Tyr Ser Gly
        195                 200                 205
Val Tyr Ala Ser Val Gly Ala Leu Arg Ser Phe Ile Asp Thr Tyr Ala
    210                 215                 220
<210> 68
<211> 35
<212> DNA
<213> Artificial Sequence
<220>
<223> artificial sequence
<400> 68
ttggaatttc ttgtggacca gcctcagcct ctctc 35
<210> 69
<211> 35
<212> DNA
<213> Artificial Sequence
<220>
<223> artificial sequence
<400> 69
gagagaggct gaggctggtc cacaagaaat tccaa 35
<210> 70
<211> 35
<212> DNA
<213> Artificial Sequence
<220>

Page 12 of 13
<223>  artificial sequence
<400>  70
tatttggaat ttcttgagca gcagcctcag cctct                                35
<210>  71
<211>  35
<212>  DNA
<213>  Artificial Sequence
<220>
<223>  artificial sequence
<400>  71
agaggctgag gctgctgctc aagaaattcc aaata                                35
<210>  72
<211>  35
<212>  DNA
<213>  Artificial Sequence
<220>
<223>  artificial sequence
<400>  72
tcttatttgg aatttcagct ggagcagcct cagcc                                35
<210>  73
<211>  35
<212>  DNA
<213>  Artificial Sequence
<220>
<223>  artificial sequence
<400>  73
ggctgaggct gctccagctg aaattccaaa taaga                                35
<210>  74
<211>  35
<212>  DNA
<213>  Artificial Sequence
<220>
<223>  artificial sequence
<400>  74
cgatcttatt tggaatagct tgtggagcag cctca                                35
<210>  75
<211>  35
<212>  DNA
<213>  Artificial Sequence
<220>
<223>  artificial sequence
<400>  75
tgaggctgct ccacaagcta ttccaaataa gatcg                                35
<210>  76
<211>  35
<212>  DNA
<213>  Artificial Sequence
<220>
<223>  artificial sequence
<400>  76
caacgatctt atttggagct tcttgtggag cagcc                                35
<210>  77
<211>  35
<212>  DNA
<213>  Artificial Sequence
<220>
<223>  artificial sequence
<400>  77
ggctgctcca caagaagctc caaataagat cgttg                                35
<210>  78
<211>  35
<212>  DNA
<213>  Artificial Sequence

Page 13 of 13
<220>
<223>  Artificial sequence
<400>  78
aaccagaagc agccaaagca gcgtaaccaa tgttt                                35
<210>  79
<211>  35
<212>  DNA
<213>  Artificial Sequence
<220>
<223>  Artificial Sequence
<400>  79
aaacattggt tacgctgctt tggctgcttc tggtt                                35
<210>  80
<211>  35
<212>  DNA
<213>  Artificial Sequence
<220>
<223>  Artificial Sequence
<400>  80
aaccagaagc agccaagaaa gcgtaaccaa tgttt                                35
<210>  81
<211>  35
<212>  DNA
<213>  Artificial Sequence
<220>
<223>  Artificial Sequence
<400>  81
aaacattggt tacgctttct tggctgcttc tggtt                                35
<210>  82
<211>  477
<212>  DNA
<213>  Pichia pastoris
<400>  82
tttttgtaga aatgtcttgg tgtcctcgtc caatcaggta gccatctctg aaatatctgg      60
ctccgttgca actccgaacg acctgctggc aacgtaaaat tctccggggt aaaacttaaa     120
tgtggagtaa tggaaccaga aacgtctctt cccttctctc tccttccacc gcccgttacc     180
gtccctagga aattttactc tgctggagag cttcttctac ggcccccttg cagcaatgct     240
cttcccagca ttacgttgcg ggtaaaacgg aggtcgtgta cccgacctag cagcccaggg     300
atggaaaagt cccggccgtc gctggcaata atagcgggcg gacgcatgtc atgagattat     360
tggaaaccac cagaatcgaa tataaaaggc gaacaccttt cccaattttg gtttctcctg     420
acccaaagac tttaaattta atttatttgt ccctatttca atcaattgaa caactat        477
<210>  83
<211>  34
<212>  DNA
<213>  Artificial Sequence
<220>
<223>  Artificial Sequence
<400>  83
ataaggatcc tttttgtaga aatgtcttgg tgtc                                 34
<210>  84
<211>  35
<212>  DNA
<213>  Artificial Sequence
<220>
<223>  Artificial Sequence
<400>  84
tataggatcc atagttgttc aattgattga aatag                                35